# EUROPÄISCHE PATENTSCHRIFT

(11) **EP 3 634 947 B1**
(45) Veröffentlichungstag und Bekanntmachung des Hinweises auf die Patenterteilung: **07.09.2022**
(21) Anmeldenummer: 18728402.1
(22) Anmeldetag: 06.06.2018
(51) Int. Cl.: C07C 263/10, C07C 265/14

(54) **VERFAHREN ZUR HERSTELLUNG VON ISOCYANATEN IN DER GASPHASE**
METHOD FOR THE PRODUCTION OF ISOCYANATES IN THE GASPHASE
PROCEDE DE PRODUCTION ISOCYANATES DANS LA PHASE GAZEUSE

(30) Priorität: 08.06.2017 EP 17175067
(43) Veröffentlichungstag der Anmeldung: 15.04.2020
(73) Patentinhaber: Covestro Intellectual Property GmbH & Co. KG, 51373 Leverkusen (DE)
(72) Erfinder: ZECHLIN, Joachim, 41472 Neuss (DE); STETTEN, Thomas, 50769 Köln (DE)
(74) Vertreter: Levpat
(86) Internationale Anmeldenummer: PCT/EP2018/064847
(87) Internationale Veröffentlichungsnummer: WO 2018/224529

(56) Entgegenhaltungen:
- EP-A1- 1 935 875
- WO-A1-2013/029918

## Beschreibung

Die Erfindung betrifft ein Verfahren zur Herstellung von Isocyanaten durch Phosgenierung der korrespondierenden primären Amine in der Gasphase, bei dem das rohe gasförmige Reaktionsproduktgemisch durch Inkontaktbringen mit mindestens einem Strom einer Quenchflüssigkeit abgekühlt und teilweise kondensiert wird, und Verwendung eines Teils des kondensierten Reaktionsproduktgemisches nach weiterer Abkühlung als Bestandteil, gegebenenfalls alleinigen Bestandteil, des mindestens einen Stroms der Quenchflüssigkeit, wobei die insgesamt eingesetzte Quenchflüssigkeit, bezogen auf ihre Gesamtmasse, organische Lösungsmittel in einem Anteil von maximal 25 Massen-% umfasst, wobei der Rest zu 100 Massen-% aus mindestens dem herzustellenden Isocyanat besteht, wobei ferner die unter dem Einfluss der Quenchflüssigkeit nicht kondensierten Anteile des Reaktionsproduktgemisches in einer Auswaschkolonne von Isocyanat-Anteilen befreit werden, wobei ein Auswaschflüssigkeit und Isocyanat umfassender Flüssigkeitsstrom mit einem auf seine Gesamtmasse bezogenen Anteil an organischen Lösungsmitteln von 0,0 Massen-% bis 10 Massen-%, insbesondere von 0,0 Massen-% bis 5,0 Massen-%, erhalten wird.

Die Herstellung von Isocyanaten, insbesondere Diisocyanaten, in der Gasphase ist schon seit längerem im Stand der Technik beschrieben und wird industriell insbesondere zur Herstellung von Toluylendiisocyanat, Xylylendiisocyanat, 1,5-Pentandiisocyanat, 1,6-Hexamethylendiisocyanat, Isophorondiisocyanat und Diisocyanatodicyclohexylmethan genutzt. In allen Verfahren fällt ein gasförmiges Rohprodukt an, das mindestens Isocyanat, Chlorwasserstoff und gegebenenfalls nicht umgesetztes Phosgen (Phosgen wird im Allgemeinen im Überschuss eingesetzt) umfasst, und das weiter aufgearbeitet werden muss, um das gewünschte Isocyanat in Reinform zu gewinnen.

Ein solches Verfahren ist beispielsweise in EP 0 289 840 B1 beschrieben. Die in einem Rohrreaktor gebildeten Diisocyanate sind bei den Reaktionstemperaturen von bis zu 500 °C thermisch nicht stabil. Eine rasche Abkühlung der Reaktionsgase nach der Phosgenierungsreaktion auf Temperaturen unter 150 °C ist daher notwendig, um die Bildung unerwünschter Nebenprodukte durch den thermischen Zerfall von Diisocyanat oder durch eine Weiterreaktion zu vermeiden. In EP 0 289 840 B1 oder EP 0749 958 B1 wird dazu das den Reaktionsraum kontinuierlich verlassende gasförmige Gemisch, welches u. a. Diisocyanat, Phosgen und Chlorwasserstoff enthält, in ein inertes Lösungsmittel, z. B. Dichlorbenzol, eingeleitet.

Weiterhin sind Verfahren bekannt, die zur Abkühlung der Reaktionsgase Wärmeaustauscher einsetzen und/oder die Gase im Vakuum entspannen (DE 101 58 160 A1).

Im Stand der Technik ist es heute bevorzugt, die Abkühlung der Reaktionsgase durch Eindüsen von unter den Reaktionsbedingungen inerten Flüssigkeiten unter möglichst vollständiger Kondensation des gebildeten Isocyanats zu bewirken. Dies geschieht in einer sog. *Abkühl- oder Quenchzone.* Ziel ist es, während der Abkühlung eine Weiterreaktion in der Flüssigphase zu unerwünschten Nebenprodukten (wie zum Beispiel Isocyanurate, Biurete, Allophanate, Carbodiimide oder Harnstoffe) weitgehend zu unterbinden.

Im Verfahren gemäß WO 2005/123665 A1 wird versucht, die Verweilzeit zwischen Reaktionsende und Abkühlzone dadurch zu verringern, dass sich zwischen der Reaktionszone und der Zone, in der der Reaktionsabbruch herbeigeführt wird, ein Bereich mit reduziertem Strömungsquerschnitt befindet. Als einzudüsende Flüssigkeiten eignen sich Lösungsmittel, Isocyanat oder Gemische aus Lösungsmittel und Isocyanat.

Auch in WO 2011/003532 A1 wird ein Verfahren zum raschen Abkühlen des gasförmigen Reaktionsgemisches mittels Eindüsen einer Quenchflüssigkeit in das kontinuierlich aus der Reaktionszone in die nachgeschaltete Quenchzone strömende Gasgemisch offenbart. Als geeignete Quenchflüssigkeiten werden Lösungsmittel, Isocyanat oder Lösungsmittel-Isocyanat-Gemische genannt.

Das Eindüsen von Quenchflüssigkeit mit Hilfe von mindestens zwei Sprühdüsen, welche am Eingang der Quenchzone angeordnet sind, wird in EP 1 403 248 A1 offenbart. Hierbei eignen sich als Quenchflüssigkeiten organische Lösungsmittel oder eine Mischung verschiedener organischer Lösungsmittel, welche mit dem gebildeten Diisocyanat nicht reagieren. Eine Lösung des gebildeten Diisocyanats in einem geeigneten organischen Lösungsmittel kann auch verwendet werden, was die Menge von eingesetztem Lösungsmittel reduziert. In diesem Fall beträgt der Volumenanteil des Lösungsmittels an der Quenchflüssigkeit von 40 bis 90 %, d. h. der Volumenanteil des Isocyanats beträgt bis zu 60 %. Der Durchmesser der Quenchzone kann größer oder kleiner sein als der Durchmesser der Reaktionszone.

Optimiert wird dieses System gemäß der Lehre von EP 1 935 875 A1 dadurch, dass zum Abbruch der Reaktion das Reaktionsgemisch aus dem Reaktionsraum heraus durch eine Kühlstrecke geführt wird, in die hinein in zwei Zonen Flüssigkeiten eingedüst werden, sodass die direkte Kühlung in der Kühlstrecke unter Erhalt nur eines Kondensationsgemisches in zwei oder mehreren hintereinander geschalteten Kühlzonen erfolgt. Das erzeugte Diisocyanat wird dabei in einem gemeinsamen Kondensationsgemisch erhalten. Dieses Gemisch wird vorzugsweise in einem Flüssigkeitssammelbehälter, angeordnet unterhalb der Kühlstrecke, aufgefangen. Dieses Kondensationsgemisch kann zur Abtrennung des hergestellten Isocyanats ausgeschleust oder, bevorzugt nach erfolgter Kühlung, teilweise auf eine oder mehrere Kühlzonen der Kühlstrecke zurückgeführt werden. Der auf diese Weise zurückgeführte Strom enthält insbesondere 30 bis 90 Gew.-% Lösungsmittel und 10 bis 70 Gew.-% Isocyanat. Neben dem Kondensationsgemisch im Sammelbehälter wird nach der Kühlstrecke ein Gasstrom, enthaltend wenigstens Chlorwasserstoff, Phosgen, gegebenenfalls Lösungsmittel, und das hergestellte Isocyanat, erhalten. Dieser Gasstrom wird dem Sammelbehälter entnommen und einer Waschkolonne zugeführt und dort weitgehend von seinen Isocyanatanteilen befreit. Bevorzugt erfolgt diese Wäsche im Gegenstrom mit Lösungsmittel. Die so erhaltene Waschphase, bestehend aus Diisocyanat und zum überwiegenden Teil aus Lösungsmittel, wird in einer bevorzugten Ausführungsform als Quenchflüssigkeit der ersten Kühlzone der Kühlstrecke eingesetzt. In der/den nachgeschalteten Kühlzone(n) wird bevorzugt das Kondensationsgemisch aus dem Sammelbehälter (das 10 bis 70 Gew.-% Isocyanat und 30 bis 90 Gew.-% Lösungsmittel enthält) als Quenchflüssigkeit eingesetzt.

In EP 1 935 876 A1 wird ebenfalls der Einsatz verschiedener geeigneter Quenchflüssigkeitsströme erwähnt. Dabei wird auch auf den Einsatz der Waschflüssigkeit aus der Gaswäsche der den Kondensatsammelbehälter nach der Quenche verlassenden Brüden als Quenchflüssigkeit hingewiesen. Ferner wird die Möglichkeit, Isocyanat als Quenchflüssigkeit einzusetzen, erwähnt (Absatz [0032]).

Auf mehrere Kühlzonen in der Quench-Stufe weist auch EP 2 196 455 A1 hin. Auf den integrierten Verbund der Kühlzonen mehrerer Reaktoren mit einer Quench-Stufe wird hier erstmals hingewiesen. Auch hier wird die Möglichkeit, Isocyanat als Quenchflüssigkeit einzusetzen, erwähnt (Absatz [0055]).

Die Anmeldung WO2007/014936 A2, Verfahren zur Herstellung von Isocyanaten (in der Gasphase), beschreibt eine Quenchzone, in welcher durch Eindüsen einer Quenchflüssigkeit das gasförmige Rohprodukt rasch abgekühlt wird. In dieser Quenchzone wird das Reaktionsgemisch, das im Wesentlichen aus den Isocyanaten, Phosgen und Chlorwasserstoff besteht, intensiv mit der eingedüsten Flüssigkeit vermischt. Die Vermischung erfolgt derart, dass die Temperatur des Reaktionsgemisches ausgehend von 200 bis 570 °C auf 100 bis 200 °C abgesenkt wird und das im Reaktionsgemisch enthaltene Isocyanat durch Kondensation vollständig oder teilweise in die eingedüsten Flüssigkeitströpfchen übergeht, während das Phosgen und der Chlorwasserstoff im Wesentlichen vollständig in der Gasphase verbleiben. Als mögliche Quenchflüssigkeiten werden Lösungsmittel, Isocyanat und Lösungsmittel-Isocyanat-Gemische genannt. Erwähnt wird das Eindüsen einer Quenchflüssigkeit zum Abkühlen des Reaktionsgemisches und selektiven Lösen des gebildeten Diisocyanats im Lösungsmittel, wobei eine erste Trennung in eine Flüssigphase und eine Gasphase mit überwiegend Phosgen und Chlorwasserstoff als Bestandteilen erfolgt. Die beiden Phasen werden danach einer entsprechenden Aufarbeitung zugeführt. Auf Optimierungsmöglichkeiten dieses Verfahrensschrittes wird nicht eingegangen.

WO 2010/063665 A1 verweist auf ein mögliches Problem der bisher genannten Quenche-Varianten. Wird zumindest ein Teil der Quenchflüssigkeit aus dem Sammelbehälter nach der Quenche, also das flüssige rohe Produktgemisch, entnommen, besteht die Möglichkeit, dass Feststoffe enthalten sein können, die die Quench-Düsen verstopfen können. Es werden verschiedene Techniken, wie z. B. Zentrifugieren, Abdestillieren des zur Quenche vorgesehenen Flüssigkeitsanteils oder Filtrieren beschrieben. Um die Temperatur des ausgewählten Quenche-Stromes für die gestellte Aufgabe einzustellen, kann der Strom mittels eines Wärmeaustauschers gekühlt, bzw. aufgeheizt werden. Diese Schrift offenbart (S. 12, Z. 6 bis 9, S. 13, zweiter Absatz) verschiedene Quellen für das Quenchmedium: Einen aus dem der Quenche 3 nachgeschalteten Phasentrenner 9 abgezweigten Teilstrom 15 (der notwendigerweise auch in der Quenche verflüssigtes Isocyanat enthält), frisches Lösungsmittel 19, einen Teil der im Phasentrenner gewonnenen Flüssigphase 13 sowie einen Teilstrom des zweiphasigen Produktstroms 7.

In WO 2010/115908 A2 wird eine spezielle Ausführungsform der Quenche offenbart. Um Folgereaktionen des Reaktionsgases bei bzw. nach der Quenchestufe zu verhindern, werden die Quenchedüsen und deren Anordnung derart konzipiert, dass eine weitgehend vollständige Benetzung der Wandung im Quenchebereich erfolgt. Damit wird das gesamte Reaktionsgemisch erfasst. Als Quenchflüssigkeiten werden Lösungsmittel sowie Gemische mit Isocyanat bzw. Rohgemisch der Phosgenierreaktion, gegebenenfalls nach Partikelentfernung, vorgeschlagen. Der Anteil an Isocyanat in der Flüssigkeit, mit der die Wandungen benetzt werden bzw. im Quenchmedium kann im Bereich von 0 bis 100 Prozent liegen (S. 11, Z. 5 bis 8).

EP 2 463 273 A1 offenbart eine Prozessvariante für Isocyanatkonzentrationen von größer als 70 Massen-% im die Quenchzone verlassenden flüssigen Sumpfprodukt. Der die Quenchzone gasförmig verlassende Strom wird ohne Durchlaufen einer Waschkolonne direkt in einen mantelgekühlten Kondensator geleitet. Der verbleibende Gasstrom wird direkt der Phosgenrückgewinnung zugeführt. Trotz der hohen Temperatur und hohen Isocyanatkonzentration im flüssigen Sumpfprodukt der Quenchzone werden über Isocyanat-Restgehalte im verbleibenden Gasstrom keine Aussagen gemacht. Der Kondensatstrom wird mit dem Kondensat des Dampfstroms, der durch Entspannung des flüssigen Sumpfproduktes aus der Quenchzone entsteht, vereinigt und als Quenchflüssigkeit zurückgeführt.

WO 2013/029918 A1 beschreibt ein Verfahren zur Herstellung von Isocyanaten durch Umsetzung der korrespondierenden Amine mit Phosgen unter anderem in der Gasphase. Dabei soll das Verfahren auch bei unterschiedlichen Auslastungen der Gasphasenanlage ohne Probleme durchgeführt werden können, insbesondere soll auch bei Fahren der Anlage im Teillastbereich das Mischen und/oder die Reaktion in dem jeweils optimierten Verweilzeitfenster erfolgen, indem das Verhältnis von Phosgen zu Amin erhöht wird oder ein oder mehrere inerte Stoffe dem Phosgen- und/oder Aminstrom zugesetzt werden. Die Anmeldung beschreibt (Seite 9, letzter Absatz bis Seite 12, erster Absatz) in diesem Zusammenhang auch verschiedene Möglichkeiten der Durchführung einer Quenche des gasförmigen Rohprodukts (Quenche mit und ohne Lösungsmittel, Einsatz von abgekühltem Produktstrom als Quenchmedium, Einsatz eines der Quenche nachgeschalteten Kondensators). Im Hinblick auf den die Quenche gasförmig verlassenden Anteil des Rohprodukts wird dessen Wäsche mit einem Lösungsmittel offenbart (Seite 12, dritter Absatz). Die Anmeldung offenbart nicht, das diese Gaswäsche auch mit einem nur geringen Lösungsmittelanteil oder sogar ganz ohne Lösungsmittel betrieben werden kann. Da ein Gasphasenverfahren zur Herstellung von Isocyanaten, in dem der gasförmige Rohproduktstrom eine Quenche durchläuft, ohne Rückgewinnung des in der Quenche nicht kondensierten Anteils des Isocyanats nicht wirtschaftlich betrieben werden kann, ist dieser Anmeldung daher auch nicht zu entnehmen, dass die Aufarbeitung eines durch eine solche Gasphasenreaktion hergestellten Isocyanats tatsächlich lösungsmittelarm oder sogar lösungsmittelfrei durchgeführt werden kann. Die vielfältigen Vorteile einer solchen Fahrweise, wie insbesondere die Möglichkeit, den mit Isocyanat beladenen Waschstrom direkt der weiteren Aufarbeitung des in der Quenche verflüssigten Isocyanat-Rohprodukts zuzuführen sowie die Möglichkeit, auf eine eigene Lösungsmittelkolonne zur Wiedergewinnung des eingesetzten Lösungsmittels zu verzichten,) werden daher durch diese Anmeldung nicht nahegelegt.

WO 2014/122180 A1 befasst sich mit einer Verfahrensführung, welche den völligen Verzicht auf eine Rezyklierung des in der Quenchzone erhaltenen Kondensationsgemisches in die Quenche ermöglicht. Zu diesem Zweck wird die in der Quenche erhaltene Gasphase vor Einleitung in eine Waschkolonne partiell kondensiert, und das erhaltene Kondensat wird anstelle rezyklierten Kondensationsproduktes als zusätzliche Quenchflüssigkeit verwendet.

WO 2015/011070 A1 befasst sich mit der Vermeidung von Ablagerungen im Bereich der Quenche. Es wird gelehrt, die Temperatur der Wand der Reaktionszone unmittelbar oberhalb der Quenchzone auf einen Wert zu halten, der maximal 15 % unter der in Kelvin angegebenen Maximaltemperatur der Wand der Reaktionszone liegt. Als geeignete Quenchflüsskeit werden Lösungsmittel, Isocyanat und Gemische aus Lösungsmittel und Isocyanat genannt.

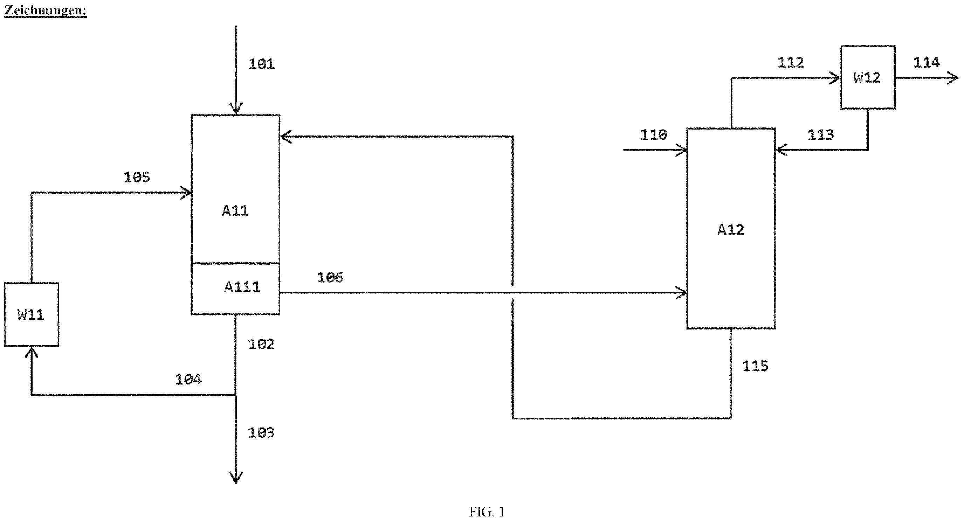

Zusammenfassend kann festgehalten werden, dass im Stand der Technik das Eindüsen von unter den Reaktionsbedingungen inerten Flüssigkeiten in den Rohproduktgasstrom die bevorzugte Variante zur raschen Abkühlung und Kondensation des Produkts darstellt. Dabei hat sich die mindestens zweistufige Quenche, in welcher in einer - in Strömungsrichtung des Produktgasgemisches betrachtet - ersten Quenchestufe eine überwiegend Lösungsmittel enthaltende Quenchflüssigkeit und in einer nachgeschalteten zweiten Quenchestufe eine zu beträchtlichen Anteilen aus dem zu bildenden Isocyanat bestehende Quenchflüssigkeit verwendet wird, als besonders vorteilhaft herausgestellt. Eine mögliche Ausführungsform dieser Verfahrensführung ist im Folgenden anhand der ersten Abbildung (FIG. 1) erläutert:
Das gasförmige Rohprodukt (101), vorwiegend bestehend aus Isocyanat, Chlorwasserstoff und überstöchiometrisch eingesetztem Phosgen, wird in der Quenche (A11) durch Eindüsen von Quenchflüssigkeit (105 und 116) schnell abgekühlt, um unerwünschte Folgereaktionen zu vermeiden. Der den Sammelbehälter (A111) im unteren Bereich der Quenche flüssig verlassende Strom (102), enthaltend vor allem Isocyanat und Quenchflüssigkeit (insbesondere 10 bis 70 Gew.-% Isocyanat und 30 bis 90 Gew.-% Lösungsmittel, vgl. EP 1 935 875 A1, Absatz [0042]), wird zum Teil zur Produktreinigung geleitet und zum Teil über den Quenchekühler (W11) als Quencheflüssigkeit zur Quenche zurückgeführt.

Der die Quenche gasförmig verlassende Stoffstrom (106), enthaltend vor allem verdampfte Quenchflüssigkeit, Chlorwasserstoff und Phosgen, wird einer Waschkolonne (A12) zugeführt, um Restgehalte an Isocyanat möglichst weitgehend aus dem Brüdenstrom zu entfernen. Je größer der Gehalt an Isocyanaten im zugeführten Stoffstrom (106) ist, desto höher muss der am Kopf der Waschkolonne zugeführte Waschflüssigkeitsstrom (Lösungsmittel) gewählt werden, und desto mehr Trennstufen werden für eine zuverlässige Rückhaltung benötigt. Der Waschflüssigkeitsstrom setzt sich aus dem Kondensat (113) des Kondensators (W12) und zusätzlichem Lösungsmittel (110) zusammen. Der praktisch Isocyanat-freie Brüdenstrom (114) enthält vor allem Phosgen und Chlorwasserstoff. Der flüssige Sumpfablauf (115) enthält vor allem (in Gehalten von 80 bis 99,99 Gew.-%; vgl. EP 1 935 875 A1, Absatz [0042]) Lösungsmittel und wird als Quenchflüssigkeit (116) der Quenche zugeführt.

Die betriebliche Erfahrung hat gezeigt, dass der Betrieb der Quenche auf diese Art und Weise, obwohl grundsätzlich möglich und vielen anderen Verfahrensführungen überlegen, nicht frei von Nachteilen ist. Insbesondere wurde, entgegen jedweder Erwartung, beobachtet, dass sich insbesondere *im Bereich der in Strömungsrichtung des Produktgasgemisches ersten, vorwiegend mit Lösungsmittel betriebenen Quenche* mit der Zeit Ablagerungen bilden, die im schlimmsten Fall eine Abschaltung und Reinigung des Reaktors erforderlich machen können. Ferner gelangen in der Reaktions- oder Quenchzone entstehende oder darin eingetragene Feststoffe sowie Schwersieder mit dem flüssigen Quencheprodukt in den Quenchekühler (W11) und die Quenchedüsen sowie unter Umständen sogar in die Auswaschkolonne (A12) und den Kondensator (W12) und können überall dort Verschmutzungen verursachen. Des Weiteren weist die Verdünnung der Isocyanat-Rohware durch die Abkühlung des gasförmigen Reaktionsproduktgemisches mit einem stark Lösungsmittel-haltigen Strom den grundsätzlichen Nachteil auf, dass das zugeführte Lösungsmittel im Zuge der weiteren Produktaufarbeitung wieder thermisch abgetrennt werden muss. Hierzu sind nicht unerhebliche Energiemengen nötig. Zudem wird bei der Eindüsung eines stark Lösungsmittel-haltigen Stroms in die Quenchezone durch die Verdampfung eines Teils des Lösungsmittels ein Brüdenstrom erzeugt, der erheblich zum Gesamtvolumen des die Quenchestufe verlassenden Gasstromes beiträgt und auf diese Weise die Abtrennung von in diesem Gasstrom mitgerissenem Isocyanat aufwändiger macht, etwa indem dieser eine größere Dimensionierung der eingesetzten Apparate bedingt. Außerdem besteht die Gefahr, dass durch die große Brüdenmenge evtl. vorhandene feste Ablagerungen aus dem Reaktor in die nachgeschaltete Abtrennung von Isocyanat aus dem in der Quenche erhaltenen gasförmigen Strom mitgerissen werden und dort zu Verstopfungen in Apparaten führen.

Aufbauend auf diesem Stand der Technik bestand daher ein Bedarf an einer weiteren Optimierung der Quenche eines gasförmigen Isocyanat-Rohprodukts. Insbesondere war es erstrebenswert, die Quenche dahingehend zu verbessern, dass Ablagerungen im Quenchebereich vermieden oder zumindest vermindert werden, und zwar ohne die Qualität und Verarbeitbarkeit des erhaltenen Kondensationsproduktes signifikant zu beeinträchtigen. Ferner war es wünschenswert, den Gehalt an Lösungsmittel im in der Quenche erhaltenen Kondensationsprodukt möglichst gering zu halten, weil das dort enthaltende Lösungsmittel in der Aufarbeitung wieder abgetrennt werden muss. Insbesondere war es daher auch wünschenswert, die Aufarbeitung möglichst einfach und damit besonders energieeffizient ausgestalten zu können.

Dem vorstehend Gesagten Rechnung tragend ist ein Gegenstand der vorliegenden Erfindung ein Verfahren zur Herstellung eines Isocyanats durch Phosgenierung des korrespondierenden primären Amins in der Gasphase umfassend die Schritte:
(i) Bereitstellung eines gasförmigen Stroms eines primären Amins;
(ii) Bereitstellung eines gasförmigen Phosgenstroms;
(iii) Vermischen des gasförmigen Stroms des primären Amins aus Schritt (i) und des gasförmigen Phosgenstroms aus Schritt (ii) zu einem gasförmigen Reaktionsgemisch unter Einhaltung eines auf primäre Aminogruppen bezogenen stöchiometrischen Überschusses an Phosgen in einer Mischzone und Führen des so erhaltenen gasförmigen Reaktionsgemisches durch eine Reaktionszone zur Umsetzung des primären Amins mit Phosgen unter Erhalt eines gasförmigen Reaktionsproduktgemisches;
(iv) Abkühlen des nach Durchlaufen der Reaktionszone aus Schritt (iii) erhaltenen gasförmigen Reaktionsproduktgemisches durch Inkontaktbringen mit mindestens einem Strom einer Quenchflüssigkeit in einer Quenchzone, wobei die insgesamt eingesetzte Quenchflüssigkeit, bezogen auf ihre Gesamtmasse, organische Lösungsmittel in einem Anteil von maximal 25 Massen-%, bevorzugt maximal 10Massen-%, besonders bevorzugt maximal 5,0 Massen-%, ganz besonders bevorzugt 0,0 Massen-%, umfasst, wobei der Rest zu 100 Massen-% aus mindestens dem herzustellenden Isocyanat besteht, unter Erhalt eines Gemisches aus Reaktionsproduktgemisch und Quenchflüssigkeit;
(v) Führen des in Schritt (iv) erhaltenen Gemisches aus Reaktionsproduktgemisch und Quenchflüssigkeit in eine Sammelzone zur Phasentrennung, wobei eine flüssige, Isocyanat umfassende Phase und eine gasförmige, Chlorwasserstoff, nicht umgesetztes Phosgen und nicht verflüssigtes Isocyanat umfassende Phase der Sammelzone entnommen werden;
(vi) Abkühlen eines Teils der in Schritt (v) der Sammelzone entnommenen, flüssigen, Isocyanat umfassenden Phase, und Einsatz der so erhaltenen abgekühlten, ansonsten jedoch nicht in ihrer Zusammensetzung veränderten, flüssigen Phase in Schritt (iv) als Bestandteil, gegebenenfalls als alleinigen Bestandteil, der Quenchflüssigkeit;
(vii) Aufarbeitung des nicht in Schritt (vi) abgekühlten und in Schritt (iv) eingesetzten Teils der in Schritt (v) der Sammelzone entnommenen flüssigen, Isocyanat umfassenden Phase zur Gewinnung des Isocyanats (d. h. zur Gewinnung des herzustellenden Isocyanats in gereinigter Form, *Rein-Isocyanat*);
(viii-1) optional, teilweises Kondensieren der in Schritt (v) erhaltenen gasförmigen, Chlorwasserstoff, nicht umgesetztes Phosgen und nicht verflüssigtes Isocyanat umfassenden Phase durch Abkühlung unter Erhalt eines flüssigen, Isocyanat umfassenden Stroms und eines gasförmigen, Chlorwasserstoff, Phosgen und nicht verflüssigtes Isocyanat umfassenden Stroms;
(viii-2) Leiten
   - entweder, bei Durchführung von Schritt (viii-1), des in Schritt (viii-1) erhaltenen gasförmigen, Chlorwasserstoff, Phosgen und nicht verflüssigtes Isocyanat umfassenden Stroms,
   - oder, wenn Schritt (viii)-1 nicht durchgeführt wird, der in Schritt (v) erhaltenen gasförmigen, Chlorwasserstoff, nicht umgesetztes Phosgen und nicht verflüssigtes Isocyanat umfassenden Phase,
   in eine Auswaschkolonne, in welcher Isocyanat mit einer Auswaschflüssigkeit unter Erhalt eines flüssigen, Auswaschflüssigkeit und Isocyanat umfassenden Stroms und eines gasförmigen, Chlorwasserstoff und Phosgen umfassenden Stroms ausgewaschen wird und der dabei erhaltene gasförmige, Chlorwasserstoff und Phosgen umfassende Strom in einem der Auswaschkolonne nachgeschalteten Kondensator (fortan als *Brüdenkondensator* bezeichnet) teilweise kondensiert wird, wobei sich die in Schritt (viii-2) insgesamt eingesetzte Auswaschflüssigkeit aus dem im Brüdenkondensator erhaltenen Kondensat und optional zusätzlich zugeführter Auswaschflüssigkeit zusammensetzt, wobei über die zusätzlich zugeführte Auswaschflüssigkeit organisches Lösungsmittel allenfalls in einer solchen Menge in die Auswaschkolonne eingetragen wird, dass sich in dem in der Auswaschkolonne erhaltenen, flüssigen, Auswaschflüssigkeit und Isocyanat umfassenden Strom ein auf dessen Gesamtmasse bezogener Anteil an organischen Lösungsmitteln von 0,0 Massen-% bis 10 Massen-%, bevorzugt von 0,0 Massen-% bis 5,0 Massen-%, einstellt.

Erfindungsgemäß weist die in Schritt (iv) *"insgesamt eingesetzte Quenchflüssigkeit, bezogen auf ihre Gesamtmasse"* einen Anteil an organischen Lösungsmitteln von *"maximal 25Massen-%, bevorzugt maximal 10 Massen-%, besonders bevorzugt maximal 5,0 Massen-%, ganz besonders bevorzugt 0,0 Massen-%"* auf. Wird in Schritt (iv) genau ein Strom an Quenchflüssigkeit eingesetzt (wird also *ausschließlich* die in Schritt (vi) nach Abkühlung erhaltene flüssige Phase als Quenchflüssigkeit eingesetzt), so muss dieser Strom diesen Anforderungen an den maximalen Lösungsmittelanteil genügen. Werden in Schritt (iv) zwei oder mehr Ströme an Quenchflüssigkeit aus unterschiedlichen Quellen mit unterschiedlicher Zusammensetzung eingesetzt, so muss der über alle in Schritt (iv) eingesetzten Ströme an Quenchflüssigkeit gemittelte Wert des Lösungsmittelanteils (also der *auf die Gesamtmasse der insgesamt eingesetzten Quenchflüssigkeit bezogene Anteil*) diesen Anforderungen genügen (bevorzugt genügt *jeder einzelne* in Schritt (iv) eingesetzte Strom an Quenchflüssigkeit den vorgenannten Anforderungen an den maximalen Lösungsmittelgehalt und besteht ansonsten aus mindestens dem herzustellenden Isocyanat). *"Organische Lösungsmittel"* (Lösungsmittel im Sinne der Erfindung sind stets *organische* Lösungsmittel) sind dabei dem Prozess zugesetzte, bei Normalbedingungen (20 °C, 1013 mbar) flüssige organische Verdünnungsmittel, die keine gegenüber Phosgen reaktive Gruppen aufweisen und in denen das herzustellende Isocyanat bei Normalbedingungen (20 °C, 1013 mbar) löslich ist, die jedoch selbstverständlich von diesem verschieden sind. Die dem Prozess zugesetzte Menge an solchen Lösungsmitteln ist bekannt; daraus und aus den ebenfalls bekannten Randbedingungen (Ort der Zugabe, Temperaturen und Drücke, Mengenströme der übrigen Einsatzstoffe) ist der Lösungsmittelanteil in der in Schritt (iv) insgesamt eingesetzten Quenchflüssigkeit für den Fachmann leicht zu ermitteln. Der nicht auf solche Lösungsmittel zurückgehende Anteil der insgesamt eingesetzten Quenchflüssigkeit besteht mindestens aus dem herzustellenden Isocyanat. Weitere Bestandteile können insbesondere hochsiedende Nebenkomponenten, die in der in Schritt (vi) erhaltenen flüssigen Phase enthalten sind, sein. Vollkommen überraschend wurde gefunden, dass der alleinige Einsatz von Quenchflüssigkeit mit einem so begrenzten Anteil an organischen Lösungsmitteln in Verbindung mit der Rezyklierung eines Teils der in der Sammelzone gewonnenen flüssigen Phase als Quenchflüssigkeit nicht - wie aufgrund des niedrigen Lösungsmittelgehaltes zu erwarten gewesen wäre - zu einer Verschlechterung der Produktqualität infolge gestiegener Nebenproduktbildung oder zur vermehrten Bildung von Ablagerungen führt, und dass dabei die Auswaschkolonne zur Rückgewinnung der die Quenche gasförmig durchlaufenden Anteile des Isocyanats lösungsmittelarm betrieben werden kann, was Vereinfachungen in der weiteren Aufarbeitung ermöglicht und den Energieverbrauch des Gesamtprozesses erheblich reduziert.

Für den Fachmann war diese Entwicklung nicht vorhersehbar, da aufgrund der ausgesprochen hohen Reaktionstemperatur in der Gasphase beim Vorliegen nur geringer Lösungsmittelkonzentrationen und damit hoher Isocyanatkonzentrationen in der eingedüsten Quencheflüssigkeit aufgrund der Thermolabilität des Isocyanats eigentlich mit Nebenreaktion zu rechnen ist. Dies gilt insbesondere in der in Strömungsrichtung des Reaktionsproduktgasgemisches ersten Quenchestufe, da hier das eingedüste Isocyanat mit einem sehr heißen, Isocyanat-haltigen Reaktionsgas in Kontakt gebracht wird.

### Es folgt zunächst eine Kurzzusammenfassung verschiedener möglicher Ausführungsformen der Erfindung:

In einer **ersten Ausführungsform** der Erfindung, die mit allen anderen Ausführungsformen kombiniert werden kann, wird Schritt (iii) adiabatisch durchgeführt, wobei Zusammensetzung und Temperatur des gasförmigen Stroms des primären Amins in Schritt (i) und des Phosgenstroms in Schritt (ii) jeweils so gewählt werden, dass sich in Schritt (iii) in der Mischzone und in der Reaktionszone eine Temperatur im Bereich von 250 °C bis 450 °C, bevorzugt im Bereich von 270 °C bis 425 °C, besonders bevorzugt im Bereich von 280 °C bis 420 °C, einstellt.

In einer **zweiten Ausführungsform** der Erfindung, die mit allen anderen Ausführungsformen kombiniert werden kann, wird die Temperatur des in Schritt (iv) eingesetzten mindestens einen Stroms an Quenchflüssigkeit auf einen Wert im Bereich von 50 °C bis 250 °C, bevorzugt von 100 °C bis 200 °C, besonders bevorzugt 120 °C bis 190 °C, eingestellt.

In einer **dritten Ausführungsform** der Erfindung, die mit allen anderen Ausführungsformen kombiniert werden kann, wird der Massenstrom der in Schritt (iv) insgesamt eingesetzten Quenchflüssigkeit so gewählt, dass dieser das 2-fache bis 60-fache, bevorzugt das 10-fache bis 50-fache, besonders bevorzugt das 20-fache bis 40-fache des Massenstroms an gasförmigem Strom des primären Amins aus Schritt (i) beträgt.

In einer **vierten Ausführungsform** der Erfindung, die mit allen anderen Ausführungsformen kombiniert werden kann, erfolgt das Inkontaktbringen des gasförmigen Reaktionsproduktgemisches mit der Quenchflüssigkeit in Schritt (iv) durch Eindüsen der Quenchflüssigkeit in das gasförmige Reaktionsproduktgemisch.

In einer **fünften Ausführungsform** der Erfindung, die eine besondere Ausgestaltung der vierten Ausfuhrungsform ist, erfolgt das Eindüsen der Quenchflüssigkeit in das gasförmige Reaktionsproduktgemisch einstufig.

In einer **sechsten Ausführungsform** der Erfindung wird der Auswaschkolonne neben dem im Brüdenkondensator erhaltenen Kondensat zusätzlich weitere Auswaschflüssigkeit zugeführt, wobei diese zusätzlich zugeführte Auswaschflüssigkeit ein Lösungsmittelstrom ist, der ausschließlich ein organisches Lösungsmittel enthält, das ausgewählt ist aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten Lösungsmittel.

In einer **siebten Ausführungsform** der Erfindung, die eine besondere Ausgestaltung der sechsten Ausführungsform ist, wird der Massenstrom des als zusätzliche Auswaschflüssigkeit eingesetzten Lösungsmittelstroms so eingestellt, dass dieser im Bereich von 20 % bis 100 %, bevorzugt im Bereich von 50 % bis 100 %, des Massenstroms des gasförmigen Stroms des primären Amins aus Schritt (i) liegt.

In einer **achten Ausführungsform** der Erfindung wird der in der Auswaschkolonne erhaltene, flüssige, Auswaschflüssigkeit und Isocyanat umfassende Strom der Aufarbeitung in Schritt (vii) zugeführt (d. h. direkt, ohne Rezyklierung in die Quenchzone aus Schritt (iv)).

In einer **neunten Ausführungsform** der Erfindung, die mit allen Ausführungsformen, welche den Schritt (viii-1) umfassen, kombiniert werden kann (mit Ausnahme der weiter unten geschilderten elften Ausführungsform), umfasst die in Schritt (iv) eingesetzte Quenchflüssigkeit zusätzlich zu der in Schritt (vi) erhaltenen abgekühlten, ansonsten jedoch nicht in ihrer Zusammensetzung veränderten, flüssigen Phase, den in Schritt (viii-1) erhaltenen flüssigen, Isocyanat umfassenden Strom.

In einer **zehnten Ausführungsform** der Erfindung, die mit allen Ausführungsformen (mit Ausnahme der nachfolgend geschilderten elften Ausführungsform) kombiniert werden kann, umfasst die in Schritt (iv) eingesetzte Quenchflüssigkeit zusätzlich zu der in Schritt (vi) erhaltenen abgekühlten, ansonsten jedoch nicht in ihrer Zusammensetzung veränderten, flüssigen Phase, den in Schritt (viii-2) erhaltenen flüssigen, Auswaschflüssigkeit und Isocyanat umfassenden Strom.

In einer **elften Ausführungsform** der Erfindung, die eine Alternative zur neunten und zehnten Ausführungsform ist, enthält die in Schritt (iv) eingesetzte Quenchflüssigkeit neben der in Schritt (vi) erhaltenen abgekühlten, ansonsten jedoch nicht in ihrer Zusammensetzung veränderten, flüssigen Phase *keine weiteren Bestandteile.*

In einer **zwölften Ausführungsform** der Erfindung, die mit allen anderen Ausführungsformen kombiniert werden kann, wird zur Gewinnung des Isocyanats in Schritt (vii) die flüssige, Isocyanat umfassende Phase ohne zwischengeschaltete Lösungsmittelkolonne (d. i. eine Destillationskolonne zur Abtrennung von Lösungsmittel) einer destillativen Reinigung zugeführt.

In einer **dreizehnten Ausführungsform** der Erfindung, die eine besondere Ausgestaltung der zwölften Ausführungsform ist, wird die destillative Reinigung in einer einzigen Destillationskolonne, die insbesondere als Trennwandkolonne ausgestaltet ist, durchgeführt.

In einer **vierzehnten Ausführungsform** der Erfindung, die mit allen anderen Ausführungsformen kombiniert werden kann, umfasst jeder einzelne in Schritt (iv) eingesetzte Strom an Quenchflüssigkeit, bezogen auf seine Gesamtmasse, organische Lösungsmittel in einem Anteil von maximal 25 Massen-%, bevorzugt maximal 10 Massen-%, besonders bevorzugt maximal 5,0 Massen-%, ganz besonders bevorzugt 0,0 Massen-%.

In einer **fünfzehnten Ausführungsform** der Erfindung, die mit allen anderen Ausführungsformen kombiniert werden kann, wird das primäre Amin ausgewählt aus der Gruppe bestehend aus Toluylendiamin, Diphenylmethandiamin, Xylylendiamin, 1,5-Pentandiamin, 1,6-Hexamethylendiamin, Isophorondiamin, Diaminodicyclohexylmethan und Mischungen der vorgenannten Verbindungen, wobei Toluylendiamin (TDA) besonders bevorzugt ist.

In einer **sechzehnten Ausführungsform** der Erfindung, die mit allen Ausführungsformen, die nicht völlig auf den Einsatz organischer Lösungsmittel verzichten, kombiniert werden kann, wird in allen Schritten, in denen ein organisches Lösungsmittel eingesetzt wird, das gleiche organische Lösungsmittel eingesetzt, das insbesondere ausgewählt wird aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel.

In einer **siebzehnten Ausführungsform** der Erfindung, die eine besondere Ausgestaltung der sechzehnten Ausführungsform ist, wird als organisches Lösungsmittel ortho-Dichlorbenzol, und zwar insbesondere in Verbindung mit TDA als dem zu phosgenierenden primären Amin, eingesetzt.

Die zuvor kurz geschilderten Ausführungsformen und weitere mögliche Ausgestaltungen der Erfindung werden im Folgenden näher erläutert. Verschiedene Ausführungsformen sind, sofern sich aus dem Kontext für den Fachmann nicht eindeutig das Gegenteil ergibt, beliebig miteinander kombinierbar.

In **Schritt** (i) wird ein Strom eines primäre Amins in gasförmiger Form bereitgestellt.

Geeignete *primäre Amine* für die Durchführung dieses Schrittes sind solche, die sich unzersetzt verdampfen lassen, insbesondere Toluylendiamin (nachfolgend TDA, insbesondere *meta*-TDA), Diphenylmethandiamin (oft auch Methylendiphenyldiamin genannt, nachfolgend MDA), Xylylendiamin (XDA, insbesondere *meta*-XDA), 1,5-Pentandiamin (nachfolgend PDA), 1,6-Hexamethylendiamin (nachfolgend HDA), Isophorondiamin (nachfolgend IDPA) und Diaminodicyclohexylmethan (nachfolgend H12-MDA). Sofern die genannten Amine in verschiedenen isomeren Formen vorliegen können, ohne dass diese angegeben sind, sind alle Isomerenverteilungen umfasst. Prinzipiell können auch Gemische der vorgenannten Amine umgesetzt werden, obwohl dies im Allgemeinen nicht bevorzugt ist.

Besonders bevorzugt ist TDA. Üblicherweise umfasst TDA, welches bevorzugt eingesetzt wird, 78,0 Massen-% bis 82,0 Massen-% 2,4-TDA und 18,0 Massen-% bis 22,0 Massen-% 2,6-TDA, bezogen auf die Gesamtmasse der 2,4- und 2,6-TDA-Isomeren. Bezogen auf die Gesamtmasse des TDA machen dabei die 2,4- und 2,6-TDA-Isomeren bevorzugt in Summe von 95,0 Massen-% bis 100 Massen-%, besonders bevorzugt von 98,0 Massen-% bis 100 Massen-%, aus. Bevorzugt beträgt der Gehalt an TDA-Isomeren mit zueinander ortho-ständigen NH₂-Gruppen im einzusetzenden TDA weniger als 0,2 Massen-%, bezogen auf die Gesamtmasse des einzusetzenden TDA. Verfahren zur Bereitstellung von TDA mit den genannten Anforderungen sind dem Fachmann bekannt.

Methoden zur Bereitstellung eines gasförmigen Aminstroms für die Durchführung von Schritt (i) sind dem Fachmann grundsätzlich bekannt. Im Folgenden werden bevorzugte Ausführungsformen geschildert.

Die Überführung des Amins in die Gasphase kann in allen aus dem Stand der Technik bekannten Verdampfungsapparaturen erfolgen, insbesondere in einem Fallfilmverdampfer. Bevorzugt werden solche Verdampfungsapparaturen eingesetzt, bei denen ein kleiner Arbeitsinhalt mit einer hohen Umwälzleistung über einen Fallfilmverdampfer geführt wird.

In einer bevorzugten Ausführungsform des erfindungsgemäßen Verfahrens werden Verdampfungsapparaturen eingesetzt, bei denen ein kleiner Arbeitsinhalt über zumindest einen Mikrowärmeaustauscher oder Mikroverdampfer umgewälzt wird. Der Einsatz entsprechender Wärmeaustauscher für die Verdampfung von Aminen wird z. B. in EP 1 754 698 A2 offenbart. Bevorzugt werden in dem erfindungsgemäßen Verfahren die in den Absätzen [0007] bis [0008] und [0017] bis [0039] der in EP 1 754 698 A2 offenbarten Apparate eingesetzt.

Zur Minimierung der thermischen Belastung des Amins ist es unabhängig von der genauen Ausgestaltung der Verdampfungsapparatur bevorzugt, den Verdampfungsvorgang durch Zuspeisung eines Inertgases wie N₂, He, Ar oder der Dämpfe eines inerten Lösungsmittels, bevorzugt ausgewählt aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel, zu unterstützen. Wird das Amin auf diese Weise verdünnt, so ist der Einsatz eines Inertgases ausgewählt aus der Gruppe bestehend aus N₂, He, Ar und Mischungen derselben als Verdünnungsmittel unter Verzicht auf die Verwendung von Dämpfen eines inerten Lösungsmittels bevorzugt.

Weiterhin erfolgt die Verdampfung - und erforderlichenfalls Überhitzung - des Ausgangsamins (insbesondere auf eine Temperatur im Bereich von 200 °C bis 430 °C, bevorzugt 250 °C bis 420 °C, besonders bevorzugt 250 °C bis 400 °C) bevorzugt mehrstufig, um unverdampfte Tröpfchen im gasförmigen Aminstrom zu vermeiden. Insbesondere bevorzugt sind mehrstufige Verdampfungs- und Überhitzungsschritte, in denen zwischen den Verdampfungs- und Überhitzungssystemen Tröpfchenabscheider eingebaut sind und / oder die Verdampfungsapparaturen auch die Funktion eines Tröpfchenabscheiders besitzen. Geeignete Tröpfchenabscheider sind dem Fachmann bekannt. Nach Verlassen des in Strömungsrichtung letzten Überhitzers wird das auf seine Solltemperatur vorgeheizte gasförmige Amin mit einer mittleren Verweilzeit von bevorzugt 0,01 s bis 60 s, ganz besonders bevorzugt von 0,01 s bis 30 s, insbesondere bevorzugt 0,01 s bis 15 s, der der Vermischung und Umsetzung in Schritt (iii) zugeführt. Unabhängig von der Ausgestaltung der Zuführung des Amins im Detail wird bevorzugt über technische Maßnahmen, z. B. eine ausreichende Isolierung zur Vermeidung von Abstrahlungsverlusten, der Gefahr einer erneuten Tröpfchenbildung begegnet.

In **Schritt** (ii) wird ein gasförmiger Phosgenstrom bereitgestellt.

Bei dem erfindungsgemäßen Verfahren wird Phosgen im Überschuss bezüglich der umzusetzenden Aminogruppen eingesetzt. Bevorzugt wird ein molares Verhältnis von Phosgen zu Amingruppen von 1,1 : 1 bis 20 : 1, besonders bevorzugt 1,2 : 1 bis 5,0 : 1 eingestellt. Auch das Phosgen wird bevorzugt, wie zuvor für das Amin beschrieben, auf eine Temperatur im Bereich von 200 °C bis 430 °C, bevorzugt 250 °C bis 420 °C, besonders bevorzugt 250 °C bis 400 °C, erhitzt und gegebenenfalls verdünnt mit einem Inertgas wie N₂, He, Ar oder mit den Dämpfen eines inerten Lösungsmittels, bevorzugt ausgewählt aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel der Vermischung und Umsetzung in Schritt (iii) zugeführt. Wird das Phosgen verdünnt, so ist der Einsatz eines Inertgases ausgewählt aus der Gruppe bestehend aus N₂, He, Ar und Mischungen derselben als Verdünnungsmittel unter Verzicht auf die Verwendung von Dämpfen eines inerten Lösungsmittels bevorzugt.

In **Schritt** (iii) werden die Reaktionspartner Amin und Phosgen vermischt und umgesetzt.

Die getrennt aufgeheizten Reaktionspartner Amin und Phosgen werden bevorzugt über eine Düsenanordnung der Vermischung und Umsetzung in Schritt (iii) zugeführt. Die Düsenanordnung zur Einführung der Eduktgasströme Amin und Phosgen kann auf verschiedene dem Fachmann bekannte Arten ausgestaltet sein; Beispiele finden sich z. B. in EP 2 199 277 B1, Abschnitt [0017] bis [0019], EP 1 449 826 B1, Abschnitt [0011] bis [0012], EP 1 362 847 B1, Abschnitt [0011] bis [0012], EP 1 526 129 B1, Abschnitt [0009] bis [0011] und EP 1 555 258 B1, Abschnitt [0008] bis [0011].

Neben der bereits erwähnten Möglichkeit, den gasförmigen Strom des primären Amins und den gasförmigen Phosgenstrom zu verdünnen, kann auch ein separater Verdünnungsgasstrom (ein Inertgas wie N₂, He, Ar oder die Dämpfe eines inerten Lösungsmittels, bevorzugt ausgewählt aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel) direkt in die Vermischung in Schritt (iii) gefahren werden. In diesem Fall wird dieser Verdünnungsgasstrom bevorzugt auf eine Temperatur im Bereich von 100 °C bis 500 °C, bevorzugt 150 °C bis 450 °C, besonders bevorzugt 150 °C bis 400 °C erhitzt. Wird der Mischzone aus Schritt (iii) ein solcher Verdünnungsgasstrom zugeführt, so ist der Einsatz eines Inertgases ausgewählt aus der Gruppe bestehend aus N₂, He, Ar und Mischungen derselben als Verdünnungsgas unter Verzicht auf die Verwendung von Dämpfen eines inerten Lösungsmittels bevorzugt.

Die Umsetzung in der Reaktionszone erfolgt bevorzugt adiabatisch. Adiabatische Umsetzung bedeutet, dass auf eine gezielte Abfuhr der entstandenen Reaktionswärme durch ein Wärmeträgermedium verzichtet wird. Daher spiegelt sich die Reaktionsenthalpie - abgesehen von unvermeidbaren Wärmeverlusten - quantitativ in der Temperaturdifferenz von Produkt- und Eduktgasstrom wider. Insbesondere betrifft die Erfindung auch ein Verfahren, bei welchem Schritt (iii) adiabatisch durchgeführt wird und wobei Zusammensetzung und Temperatur des gasförmigen Stroms des primären Amins in Schritt (i) und des Phosgenstroms in Schritt (ii) jeweils so gewählt werden, dass sich in Schritt (iii) in der Mischzone und in der Reaktionszone eine Temperatur im Bereich von 250 °C bis 450 °C, bevorzugt im Bereich von 270 °C bis 425 °C, besonders bevorzugt im Bereich von 280 °C bis 420 °C, einstellt. Damit ist gemeint, dass die Temperatur an jedem Punkt in der Mischzone und der Reaktionszone in diesem Bereich liegt.

Mischzone und Reaktionszone sind dabei bevorzugt in einer gemeinsamen technischen Vorrichtung zur Durchführung chemischer Reaktionen angeordnet, dem *Reaktor.* Bei dieser Anordnung gehen im Allgemeinen Misch- und Reaktionszone fließend ineinander über, ohne dass - wie bei Einsatz einer separaten Mischapparatur, was grundsätzlich auch möglich ist - eine strenge Abgrenzung beider möglich wäre. Die Reaktionszone nach Vermischung der Reaktanden dient zur Bereitstellung von Verweilzeitraum, um möglichst vollständigen Umsatz sicher zu stellen.

Der Reaktor hat bevorzugt im Bereich der Mischzone und im Bereich der Reaktionszone einen runden (insbesondere kreissymmetrischen) Querschnitt. Dabei kann der ganze Reaktor zylinderförmig sein. Es ist jedoch auch möglich, dass sich der Querschnitt verändert wie z. B. in EP 1275639 B1, Abschnitt [0009], EP 1 275 640 A1, Abschnitt [0014], EP 1 403 248 B1, Abschnitt [0014] bis [0015], EP 193 5876 A1, Abschnitt [0012] bis [0016] und EP 2 196 455 B1, Abschnitte [0015] bis [0026] und [0027] bis [0030] beschrieben. Weitere Details zum Bau geeigneter Phosgenierungsreaktoren sind dem Fachmann bekannt.

In der Reaktionszone werden Amin und Phosgen rasch zum korrespondierenden Isocyanat umgesetzt, wie beschrieben bevorzugt adiabatisch. Die Reaktion wird bevorzugt so geführt, dass das Amin vor Eintritt in die weiter unten näher beschriebene Quenchzone vollständig umgesetzt ist. In einer bevorzugten Ausführungsform des erfindungsgemäßen Verfahrens beträgt die Durchsatzkapazität des eingesetzten Reaktors unter Reaktionsbedingungen > 1,0 t Amin / h, bevorzugt 2,0 bis 50 t Amin / h, besonders bevorzugt 5,0 bis 15 t Amin / h. Besonders bevorzugt gelten diese Werte für Toluylendiamin, 1,6-Diamino-hexan und für Isophorondiamin. Unter Durchsatzkapazität ist dabei zu verstehen, dass in dem Reaktor die genannte Durchsatzkapazität an Amin pro Stunde umgesetzt werden kann. Bevorzugt strömen die Edukt- und Produktgase ohne wesentliche Rückvermischung durch den Reaktor. Dies wird durch ein Druckgefälle über die Misch- und Reaktionszone, bevorzugt über die Mischzone, Reaktionszone und die sich anschließende Quenchzone, sichergestellt. Bevorzugt besteht das Druckgefälle zwischen dem Beginn der Mischzone einerseits und dem Ausgang aus der Quenchzone andererseits. Bevorzugt liegt der absolute Druck am Beginn der Mischzone bei 200 mbar bis 3000 mbar und hinter der Quenchzone bei 150 mbar bis 2500 mbar. Wesentlich ist hierbei jedoch lediglich die Aufrechterhaltung eines Differenzdrucks über die Reaktionszone, bevorzugt über die Reaktionszone und die Quenchzone von bevorzugt mindestens 40 mbar zwecks Gewährleistung der genannten gerichteten Strömung und einer guten Vermischung der Edukte.

In **Schritt** (iv) erfolgt die rasche Abkühlung und weitgehende Verflüssigung des gebildeten Isocyanats ("Quenche").

Die erfindungsgemäße Forderung, dass *"die insgesamt eingesetzte Quenchflüssigkeit, bezogen auf ihre Gesamtmasse, organische Lösungsmittel in einem Anteil von maximal 25 Massen-%, bevorzugt maximal 10 Massen-%, besonders bevorzugt maximal 5,0 Massen-%, ganz besonders bevorzugt 0,0 Massen-%, umfasst"* bedeutet, dass im Rahmen der vorliegenden Erfindung der im Stand der Technik übliche Einsatz von Quenchflüssigkeit mit einem hohen Lösungsmittelgehalt (insbesondere Quenchflüssigkeit mit 30 bis 90 Massen-% Lösungsmittel aus dem Sumpfprodukt der Kühlstrecke und/oder sogar 80 bis 99,99 Massen-% Lösungsmittel aus der Gaswäsche; vgl. EP 1 935 875 A1, Absatz [0042]) *nicht angewandt wird.* Sofern die in Schritt (iv) eingesetzte Quenchflüssigkeit ein organisches Lösungsmittel enthält, ist dieses bevorzugt ausgewählt aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel. Nach der erfolgten Phosgenierungsreaktion in der Reaktionszone wird das gasförmige Reaktionsproduktgemisch, das wenigstens das herzustellende Isocyanat, Chlorwasserstoff und nicht umgesetztes (da überstöchiometrisch eingesetzt) Phosgen umfasst, in die *Quenchzone* geleitet, wo das gebildete Isocyanat durch Inkontaktbringen mit, d. h. insbesondere durch Eindüsen von, Quenchflüssigkeit abgekühlt und so (weitgehend) kondensiert wird. Möglichkeiten für den Aufbau und den Betrieb einer Quenchzone sind im Prinzip aus dem Stand der Technik bekannt. Sofern die erfindungsgemäßen Anforderungen im Hinblick auf die Zusammensetzung der Quenchflüssigkeit eingehalten werden, können die Apparate und Methoden des Standes der Technik eingesetzt werden. Mögliche Ausgestaltungen der Quenchzone sind beispielsweise in EP 1 403 248 A1 und EP 1 935 875 A1 offenbart. Einhaltung der erfindungsgemäßen Anforderungen im Hinblick auf die Zusammensetzung der Quenchflüssigkeit bedeutet, dass die im gesamten Quencheschritt (iv) insgesamt eingesetzte Quenchflüssigkeit 0,0 Massen-% bis 25,0 Massen-% an organischen Lösungsmitteln, bevorzugt 0,0 Massen-% bis 10,0 Massen-% an organischen Lösungsmitteln, besonders bevorzugt 0,0 Massen-% bis 5,0 Massen-% an organischen Lösungsmitteln enthält, jeweils bezogen auf die Gesamtmasse der insgesamt eingesetzten Quenchflüssigkeit. Außerordentlich besonders bevorzugt enthält die insgesamt eingesetzte Quenchflüssigkeit kein organisches Lösungsmittel und besteht - abgesehen von Verunreinigungen (wie z. B. hochsiedenden Nebenkomponenten in der flüssigen Phase aus Schritt (vi)), die in Anteilen von insbesondere bis zu 5,0 Massen-%, bezogen auf die Gesamtmasse der insgesamt eingesetzten Quenchflüssigkeit, vorhanden sein können - aus dem herzustellenden Isocyanat. Werden mehrere Ströme an Quenchflüssigkeit eingesetzt, so gelten die zuvor genannten Zusammensetzungsbereiche mindestens für den über alle eingesetzten Ströme an Quenchflüssigkeit gemittelten Massenanteil an organischen Lösungsmitteln, bevorzugt jedoch für jeden einzelnen Strom an Quenchflüssigkeit.

Wenn in die Gasphasenreaktion kaum noch Lösungsmittel eingebracht wird, reduziert sich weiterhin auch der Mengenstrom der in Schritt (v) anfallenden Gasphase, was eine Vereinfachung der nachgeschalteten Aufarbeitungsschritte zur Folge hat, wie später noch näher erläutert wird.

In einer bevorzugten Ausführungsform wird Schritt (iv) so ausgestaltet, dass die Quenchflüssigkeit an nur einer Position der Quenchzone in das Reaktionsproduktgemisch eingedüst wird *(einstufige* Quenche). Mit "*an nur einer Position* " ist dabei bei einer Quenchzone in einem aufrecht stehenden Reaktor mit rundem (insbesondere kreissymmetrischen) Querschnitt eine einzige Position entlang der Längsrichtung des Reaktors gemeint. Dabei kann die Quenchflüssigkeit durchaus mithilfe mehrerer Düsen *entlang des Querschnitts* der Quenchzone verteilt werden. Dies geschieht jedoch an derselben Position *entlang der Längsrichtung des Reaktors,* im Unterschied zur in FIG. 1 gezeigten Verfahrensweise mit Zufuhr von Quenchflüssigkeit an *zwei unterschiedlichen* Positionen (siehe 116 und 105) entlang der Längsrichtung des Reaktors.

Die Temperatur der in Schritt (iv) eingesetzten Quenchflüssigkeit wird bevorzugt so gewählt, dass sie einerseits hoch genug ist, um das dem Isocyanat entsprechende Carbamoylchlorid in Isocyanat und Chlorwasserstoff zurückzuspalten. (Es ist zwar keineswegs sicher, ob das aus der Flüssigphasenphosgenierung bekannte Zwischenprodukt Carbamoylclorid auch in der Gashasenphosgenierung gebildet wird. Da es jedoch unabhängig davon denkbar ist, dass in der Quenche *verflüssigtes* Isocyanat mit dem vorhandenem Chlorwasserstoffgas teilweise zum Carbamoylchlorid reagiert, sollte die Temperatur der Quenchflüssigkeit hoch genug sein, um diese Reaktion zurückzudrängen.) Andererseits sollen Isocyanat und gegebenenfalls das in dem gasförmigen Aminstrom und / oder gasförmigen Phosgenstrom als Verdünnungsmittel mit verwendete Lösungsmittel weitestgehend kondensieren bzw. sich weitestgehend in dem Lösungsmittel lösen, während überschüssiges Phosgen, Chlorwasserstoff und gegebenenfalls als Verdünnungsmittel mit verwendetes Inertgas die Quenchzone weitestgehend nicht kondensiert bzw. nicht gelöst durchlaufen, sodass die Temperatur der Quenchflüssigkeit auch nicht zu hoch gewählt werden darf. Zur selektiven Gewinnung des Isocyanats aus dem gasförmigen Reaktionsgemisch besonders gut geeignet sind insbesondere bei einer Temperatur von 50 °C bis 250 °C, bevorzugt 100 °C bis 200 °C, besonders bevorzugt 120 °C bis 190 °C, gehaltene Quenchflüssigkeiten (werden mehrere Ströme an Quenchflüssigkeit eingesetzt, so wird die Temperatur eines jeden Stroms an Quenchflüssigkeit in diesem Bereich gehalten). Dabei wird der Massenstrom an in Schritt (iv) insgesamt eingesetzter Quenchflüssigkeit bevorzugt so gewählt, dass dieser das 2-fache bis 60-fache, besonders bevorzugt das 10-fache bis 50-fache, ganz besonders bevorzugt das 20-fache bis 40-fache des Massenstroms an gasförmigem Strom des primären Amins aus Schritt (i) beträgt.

Es ist für den Fachmann aufgrund der physikalischen Daten bei gegebener Temperatur, Druck und Zusammensetzung einfach vorhersagbar, welcher Massenanteil des Isocyanats in der Quenche kondensiert bzw. diese nicht kondensiert durchläuft. Ebenso ist es einfach vorherzusagen, welcher Massenanteil des überschüssigen Phosgens, Chlorwasserstoffs, gegebenenfalls Lösungsmittel und gegebenenfalls als Verdünnungsmittel verwendeten Inertgases die Quenche unkondensiert durchläuft bzw. sich in der Quenchflüssigkeit löst. Das so in der Quenchzone erhaltene Gemisch aus Reaktionsproduktgemisch und Quenchflüssigkeit enthält daher gasförmige Anteile und flüssige Anteile, ist also zweiphasig.

Bevorzugt sind Mischzone, Reaktionszone und Quenchzone in einer gemeinsamen Vorrichtung, dem Reaktor, angeordnet. Insbesondere bevorzugt hat der Reaktor im Bereich der Mischzone, Reaktionszone und Quenchzone einen runden (insbesondere kreissymmetrischen) Querschnitt und ist entweder von zylindrischer Form oder weist - wie oben beschrieben - Abschnitte unterschiedlichen Querschnitts auf, sodass der Reaktor im Bereich der Mischzone, Reaktionszone und Quenchzone aus Zylindern unterschiedlichen Durchmessers, die über kegelförmige Übergangsstücke verbunden sind, besteht. Der Reaktor steht dabei bevorzugt aufrecht, und die Eduktgasströme Amin und Phosgen (sowie die aus diesen gebildeten Zwischen- und Endprodukte) durchlaufen den Reaktor bevorzugt *von oben nach unten.* Mischzone, Reaktionszone und Quenchzone sind dann in dieser Reihenfolge von oben nach unten in dem Reaktor angeordnet. Bevorzugt ist auch die im Folgenden noch näher geschilderte Sammelzone Bestandteil des Reaktors und ist bei aufrechter Anordnung desselben insbesondere unterhalb der Quenchzone angeordnet. Auch die Sammelzone hat bevorzugt einen runden (insbesondere kreissymmetrischen) Querschnitt; andere Ausgestaltungen sind aber denkbar.

In **Schritt** (v) wird das in Schritt (iv) anfallende Gemisch aus Reaktionsproduktgemisch und Quenchflüssigkeit in eine *Sammelzone* zur Phasentrennung geführt.

In der zuvor geschilderten bevorzugten Ausführungsform der gemeinsamen Anordnung von Mischzone, Reaktionszone, Quenchzone und Sammelzone von oben nach unten in der genannten Reihenfolge in einem aufrecht stehenden Reaktor fließt das in Schritt (iv) anfallende Gemisch aus Reaktionsproduktgemisch und Quenchflüssigkeit schwerkraftbedingt (also "automatisch") in die Sammelzone. Bei anderer Anordnung der Sammelzone ist es unter Umständen erforderlich, das Gemisch aus Reaktionsproduktgemisch und Quenchflüssigkeit in die Sammelzone zu pumpen.

In der Sammelzone findet eine Auftrennung des in Schritt (iv) erhaltenen Gemisches aus Reaktionsproduktgemisch und Quenchflüssigkeit in eine flüssige, Isocyanat und gegebenenfalls Lösungsmittel umfassende Phase und eine gasförmige, (mindestens) Chlorwasserstoff, nicht umgesetztes Phosgen und nicht verflüssigtes Isocyanat und - sofern vorhanden - verdampftes Lösungsmittel umfassende Phase statt. Die flüssige und gasförmige Phase werden der Sammelzone kontinuierlich entnommen.

In **Schritt** (vi) erfolgt die Abkühlung und Rezyklierung eines Teils der in Schritt (v) der Sammelzone entnommenen Flüssigphase.

Die Abkühlung eines Teils der der Sammelzone entnommenen Flüssigphase erfolgt indirekt, d. h. ohne direkten (stofflichen) Kontakt der der Sammelzone entnommenen Flüssigphase mit einem flüssigen Kühlmedium. Für diesen Zweck ist insbesondere ein Wärmeaustauscher geeignet. Die Abkühlung in Schritt (vi) erfolgt bevorzugt auf eine Temperatur im Bereich von 30 °C bis 245 °C, bevorzugt 80 C bis 195 °C, besonders bevorzugt 100 °C bis 195 °C, ganz besonders bevorzugt 100 °C bis 185 °C. Da diese rezyklierte Flüssigphase außer der Abkühlung keine weitere Behandlung erfährt, also nicht weiter verändert wird, entspricht ihre Zusammensetzung in dieser Ausführungsform derjenigen der Flüssigphase in der Sammelzone.

Weiterhin kann die Effizienz der Kühlung der der Sammelzone entnommenen Flüssigphase durch einen externen Wärmeaustauscher durch das erfindungsgemäße Verfahren erheblich gesteigert werden, da die Wärmekapazität der Flüssigphase infolge ihres geringen Lösungsmittelanteils vergleichsweise hoch ist. Zudem wird bei Einsatz des erfindungsgemäßen Verfahrens die Effizienz des externen Wärmeaustauschers zusätzlich gesteigert, da das nunmehr nicht oder nur in geringem Umfang mit Lösungsmittel verdünnte Isocyanat aufgrund der besseren Lösungseigenschaften für Rückstandskomponenten die Oberflächen des Wärmeaustauschers wirksam vor Ablagerungen schützen kann.

In einer Ausführungsform der Erfindung kann die in der Sammelzone vorliegende Flüssigphase bereits vor ihrer Entnahme durch Rückführung eines Kondensats aus der - weiter unten näher beschriebenen - Rückstandsaufarbeitung, das fast nur oder vollständig aus dem herzustellenden Isocyanat besteht (insbesondere zu 95 Massen-% bis 100 Massen-%, bezogen auf die Gesamtmasse des Kondensats), gekühlt werden. Diese Rückführung ermöglicht zum einen eine zusätzliche Kühlung der in der Sammelzone erhaltenen Flüssigphase unter Verringerung der abgedampften Isocyanat-Menge (also unter Verringerung der Schritt (v) gebildeten und der Sammelzone entnommenen Gasphase), zum anderen kann die Rückstandskonzentration im Sumpf des Reaktors durch Verdünnung niedrig gehalten werden. Der Kühlungseffekt geht einher mit der Aufheizung des Kondensats aus der Rückstandsaufarbeitung, was zu einer Energieeinsparung führt, da dieses Material ansonsten wieder in der Isocyanat-Destillationstufe aufgeheizt werden müsste.

In weiteren Ausführungsformen können ausreichend abgekühlte Isocyanat-Ströme aus beliebigen Prozess-Schritten in die Sammelzone zurückgeführt werden, um dort zu einer Abkühlung und Verdünnung zu führen.

Bevorzugt werden in Schritt (vi) 50 % bis 96 %, besonders bevorzugt 60 % bis 94 %, ganz besonders bevorzugt 70 % bis 92 % der in Schritt (v) der Sammelzone entnommenen Flüssigphase als Quenchflüssigkeit rezykliert, während der Rest (sog. Isocyanat-Rohware) einer Aufarbeitung zur Gewinnung des Isocyanats in gereinigter Form (Rein-Isocyanat) zugeführt wird. Das erfindungsgemäße Verfahren ermöglicht in besonders bevorzugten Ausführungsformen die Bereitstellung einer Isocyanat-Rohware mit einem Gehalt an organischen Lösungsmitteln von maximal 20 Massen-%, bevorzugt maximal 8,0 Massen-%, besonders bevorzugt maximal 4,0 Massen-%, ganz besonders bevorzugt 0,0 Massen-%, bezogen auf die Gesamtmasse der Isocyanat-Rohware.

In **Schritt** (vii) erfolgt die Aufarbeitung der restlichen, nicht als Quenchflüssigkeit rezyklierten, Flüssigphase aus der Sammelzone (Isocyanat-Rohware) zur Gewinnung (Isolierung) des Isocyanats in gereinigter Form (Rein-Isocyanat).

Bevorzugt erfolgt diese Aufarbeitung destillativ. Abhängig vom Lösungsmittelgehalt der in der Sammelzone erhaltenen Flüssigphase kann die Aufarbeitung ein- oder mehrstufig erfolgen.

Enthält die in Schritt (v) der Sammelzone entnommene flüssige, Isocyanat umfassende Phase substanzielle Anteile an Lösungsmittel (wenn sich also der Lösungsmittelgehalt eher im oberen Bereich des erfindungsgemäß zulässigen Bereichs befindet), so kann es vorteilhaft sein, diese Flüssigphase zunächst *in einem ersten Destillationsschritt **(vii-1)*** in einer sog. Lösungsmittelkolonne vom Hauptteil des Lösungsmittels zu befreien, bevor sich *in mindestens einem weiteren Destillationsschritt (vii-2)* eine Feinreinigung des Isocyanats anschließt. Die Aufarbeitung kann durchgeführt werden wie aus dem Stand der Technik bekannt, insbesondere wie in EP 1 371 633 A1 beschrieben. Es ist ebenfalls möglich, dem Destillationsschritt (vii-1) eine an sich aus dem Stand der Technik bekannte separate Abtrennung von gelöstem Phosgen und gelöstem Chlorwasserstoff voranzustellen **(Schritt (vii-0))**.

Grundsätzlich kann auf den zuvor genannten ersten Destillationsschritt (vii-1) auch verzichtet werden; die Isocyanat-Rohware wird dann ohne zwischengeschaltete Lösungsmittelkolonne(n) der Feinreinigung zugeführt. Dies ist insbesondere dann vorteilhaft, wenn die in Schritt (v) der Sammelzone entnommene flüssige, Isocyanat umfassende Phase allenfalls geringe Anteile an Lösungsmittel enthält (wenn sich also der Lösungsmittelgehalt eher im unteren Bereich des erfindungsgemäß zulässigen Bereichs befindet). Bei Verzicht auf den ersten Destillationsschritt wird die in der Sammelzone anfallende Flüssigphase, insoweit nicht in die Quenche (Schritt (iv)) rezykliert, unmittelbar - oder nach allenfalls einer aus dem Stand der Technik an sich bekannten separaten Abtrennung von gelöstem Phosgen und gelöstem Chlorwasserstoff - einer Destillation zur Gewinnung von Rein-Isocyanat zugeführt. Bevorzugt erfolgt diese Destillation in einer einzigen Destillationskolonne, die insbesondere als Trennwandkolonne ausgestaltet sein kann. Neben der offensichtlichen apparativen Vereinfachung wird in dieser Ausführungsform die Energieeffizienz des Prozesses durch die eingesparte Lösungsmittel-Verdampfung in erheblichem Maße gesteigert.

Unabhängig von der genauen Ausgestaltung der destillativen Aufarbeitung in Schritt (vii) fällt neben dem (als Destillatstrom) gewonnenen Rein-Isocyanat auch mindestens ein *Destillationssumpfstrom* an (beispielsweise der Destillationssumpfstrom der in einer bevorzugten Ausführungsform der Erfindung in Schritt (vii-2) eingesetzten Trennwandkolonne). Dieser Destillationssumpfstrom enthält den sog. Destillationsrückstand und Anteile des herzustellenden Isocyanats.

Der Destillationsrückstand enthält solche Verbindungen, die unter den für die Destillation gewählten Bedingungen von Druck und Temperatur nicht verdampfen oder die sich überhaupt nicht unzersetzt verdampfen lassen. Die schwer oder gar nicht verdampfbaren Verbindungen im Destillationsrückstand sind - sofern es sich nicht um den Phosgenierungsprozess unverändert durchlaufende Verunreinigungen aus dem eingesetzten primären Amin handelt - höhermolekulare Phosgenierungsprodukte, deren Struktur nicht immer exakt bekannt ist. So kann es sich um Verbindungen handeln, die sich (formal) von Polymerisationsprodukten des eingesetzten Amins durch Ersatz der nicht polymerisierten Amin- durch Isocyanat-Gruppen ableiten lassen. Höhermolekulare Phosgenierungsprodukte können sich zum Teil (durch Weiterreaktion) auch noch in Schritt (vii) bilden.

Der den Destillationsrückstand enthaltende Destillationssumpfstrom wird bevorzugt ebenfalls aufgearbeitet **(Schritt** vii-3)), wobei diese Aufarbeitung folgende Schritte umfasst:
a) optionale Vorkonzentrierung des Destillationssumpfstroms in einem Verdampfer durch partielle Verdampfung des in dem Destillationssumpfstrom enthaltenen herzustellenden Isocyanats, wobei ein an herzustellendem Isocyanat abgereicherter vorkonzentrierter flüssiger Strom erhalten wird;
b) Trocknung des Destillationssumpfstroms oder des in Schritt a) erhaltenen vorkonzentrierten, an herzustellendem Isocyanat abgereicherten, flüssigen Stroms in einer Trocknungsvorrichtung bei einer Temperatur im Bereich von 150 °C bis 500 °C, bevorzugt im Bereich von 185 °C bis 300 °C, besonders bevorzugt im Bereich von 200 °C bis 270 °C, und bei einem Druck im Bereich von 10 mbar_{(abs.)} bis 250 mbar_{(abs.)}, bevorzugt im Bereich von 20 mbar_{(abs.)} bis 200 mbar_{(abs.)}, besonders bevorzugt im Bereich von 30 mbar_{(abs.)} bis 100 mbar_{(abs.)} durchgeführt wird, wobei herzustellendes Isocyanat unter Bildung eines festen Verfahrensprodukts verdampft und zurückgewonnen wird.

Die optionale Vorkonzentrierung durch partielle Verdampfung gemäß Schritt a) kann grundsätzlich in allen dem Fachmann bekannten Verdampfern erfolgen. Besonders bevorzugt wird Schritt a) in einem Verdampfer ausgewählt aus der Gruppe bestehend aus Dünnschichtverdampfern, Kletterverdampfern, Fallfilmverdampfern, Langrohrverdampfern, Wendelrohrverdampfern, Zwangsumlaufentspannungsverdampfer und einer Kombination dieser Apparate durchgeführt. Dabei sind Fallfilmverdampfer besonders bevorzugt. Es ist auch möglich, mehrere Verdampfer in Serie zu schalten. Bevorzugt erfolgt die Vorkonzentrierung gemäß Schritt a) bei einer Temperatur im Bereich von 120 °C bis 180 °C und bei einem Druck im Bereich von 20 mbar_{(abs.)} bis 60 mbar_{(abs.)}, besonders bevorzugt bei einer Temperatur im Bereich von 130 °C bis 175 °C und bei einem Druck im Bereich von 25 mbar_{(abs.)} bis 45 mbar_{(abs.)}. Schritt a) kann kontinuierlich oder diskontinuierlich durchgeführt werden. Die kontinuierliche Verfahrensführung ist bevorzugt.

In Schritt b) erfolgt die Trocknung des in Schritt a) erhaltenen, an herzustellendem Isocyanat abgereicherten, vorkonzentrierten flüssigen Stroms bzw. - bei Verzicht auf Schritt a) - des in Destillationssumpfstromes. Für den Schritt b) geeignete Trocknungsvorrichtungen sind bevorzugt ausgewählt aus der Gruppe bestehend aus beheizten, produktumschaufelnden Vakuumtrocknern mit horizontaler Welle (bevorzugt Knetertrockner, Paddeltrockner, Schaufeltrockner; wobei jeder der genannten Trockner genau eine Welle oder mehrere Wellen, insbesondere zwei Wellen, aufweisen kann), Drehrohren, Scheibentrocknern, Bandtrocknern und Granulierschnecken. Bei der Trocknung wird das herzustellende Isocyanat verdampft und zurückgewonnen. Es verbleibt ein Feststoff, der nahezu ausschließlich aus Destillationsrückstand besteht und das herzustellende Isocyanat allenfalls noch in Spuren enthält. Der Feststoff wird bevorzugt kontinuierlich aus der Trocknungsvorrichtung ausgetragen.

Das in Schritt a) oder in den Schritten a) und b) verdampfte Isocyanat wird kondensiert. Bei Durchführung von Schritt a) und b) werden die erhaltenen Kondensate bevorzugt vereinigt. Der auf diese Weise zurückgewonnene Anteil des herzustellenden Isocyanats wird bevorzugt teilweise bis vollständig, bevorzugt vollständig, mit dem Rein-Isocyanat vereinigt oder an eine andere Stelle des Verfahrens zurückgeführt. Es ist beispielsweise möglich, den in Schritt (vii-3) gewonnenen Anteil des herzustellenden Isocyanats in Schritt (vii-2) zurückzuführen, insbesondere in den Zulauf oder in den Destillationssumpfstrom der dort eingesetzten Destillationskolonne (bei mehreren in Serie geschalteten Destillationskolonnen bevorzugt in den Zulauf oder in den Destillationssumpfstrom der letzten Destillationskolonne). In einer anderen Ausführungsform wird das erhaltene Isocyanat-Kondensat, wie bereits weiter oben beschrieben, zur direkten (Vor-)Kühlung der in der Sammelzone enthaltenden Flüssigphase verwendet.

Erfindungsgemäß wird auch die in Schritt (v) der Sammelzone entnommene *gasförmige,* Chlorwasserstoff, nicht umgesetztes Phosgen und nicht verflüssigtes Isocyanat und - sofern vorhanden - verdampftes Lösungsmittel umfassende *Phase* aufgearbeitet **(Schritt** (viii)).

In dieser weiteren Aufarbeitung wird insbesondere nicht verflüssigtes Isocyanat zurückgewonnen und es werden Lösungsmittel (sofern in der in Schritt (v) der Sammelzone entnommenen Gasphase vorhanden), überschüssiges Phosgen und entstandenes Chlorwasserstoffgas voneinander getrennt. Das zurückgewonnene Isocyanat wird in den Prozess zurückgeführt, wie im Folgenden noch näher erläutert wird. Auch das Lösungsmittel (sofern in substanziellen Anteilen vorhanden) wird aus wirtschaftlichen Gründen bevorzugt wieder dem Prozess zugeführt. Insbesondere ist es bevorzugt, das zurückgewonnene Lösungsmittel als Bestandteil der Quenchflüssigkeit einzusetzen, wie weiter unten noch näher erläutert wird. Phosgen wird aus wirtschaftlichen Gründen ebenfalls bevorzugt wieder der Reaktion zugeführt, und zwar insbesondere dem Schritt (i). Der zurückgewonnene Chlorwasserstoff kann verschiedenen Einsatzmöglichkeiten zugeführt werden, wie zum Beispiel einer Oxychlorierung von Ethylen zu Ethylendichlorid oder einem Recyclingverfahren, welches Chlor, das wieder in den Isocyanatprozess zurückgeführt werden kann, liefert. Zu diesen Recyclingverfahren zählen die katalytische Oxidation von Chlorwasserstoff, z. B. nach dem Deacon-Verfahren, die Elektrolyse von gasförmigem Chlorwasserstoff sowie die Elektrolyse einer wässrigen Lösung von Chlorwasserstoff (Salzsäure), erhalten durch Absorption in Wasser des gebildeten Chlorwasserstoffs.

In Schritt (viii) wird zunächst nicht verflüssigtes Isocyanat aus der in Schritt (v) der Sammelzone entnommenen gasförmigen Phase zurückgewonnen.

Dies kann in einer Ausführungsform so geschehen, dass man zunächst die in Schritt (v) erhaltene gasförmige, (mindestens) Chlorwasserstoff, nicht umgesetztes Phosgen und nicht verflüssigtes Isocyanat und - sofern vorhanden - verdampftes Lösungsmittel umfassende Phase in einem **Schritt (viii-1)** durch Abkühlung (d. h. indirekte Abkühlung [also ohne stofflichen Kontakt mit einem flüssigen Wärmeträgermedium]; diese Abkühlung wird bevorzugt in einem *Wärmeaustauscher* durchgeführt) unter Erhalt eines flüssigen, Isocyanat und gegebenenfalls Lösungsmittel umfassenden Stroms und eines gasförmigen, Chlorwasserstoff, Phosgen, nicht verflüssigtes Isocyanat und gegebenenfalls nicht verflüssigtes Lösungsmittel umfassenden Stroms teilweise (d. h. die Verflüssigung von *Lösungsmittel* - sofern vorhanden - *und Isocyanat* erfolgt bewusst nicht vollständig) kondensiert.

In dieser Ausführungsform wird der so erhaltene gasförmigen, Chlorwasserstoff, Phosgen, nicht verflüssigtes Isocyanat und gegebenenfalls nicht verflüssigtes Lösungsmittel umfassende Strom zur Abtrennung von restlichem Isocyanat in **Schritt (viii-2)** in eine Auswaschkolonne geleitet und dort mit einer Auswaschflüssigkeit in Kontakt gebracht, wobei ein flüssiger, Isocyanat, Auswaschflüssigkeit und gegebenenfalls Lösungsmittel umfassender Strom und ein gasförmiger, Chlorwasserstoff und Phosgen umfassender Strom erhalten werden.

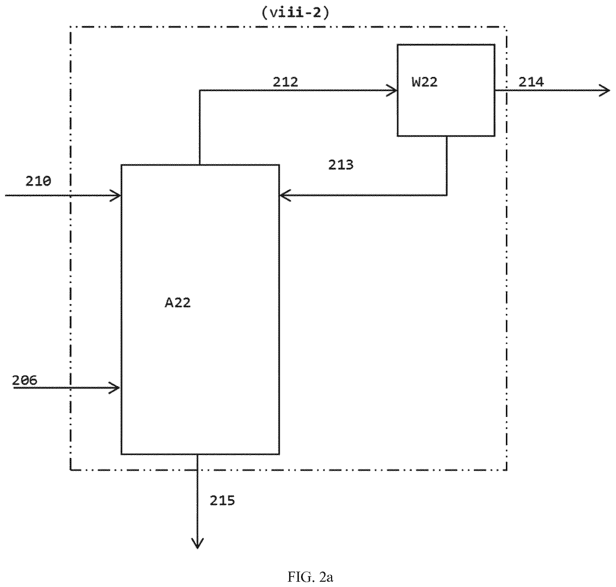

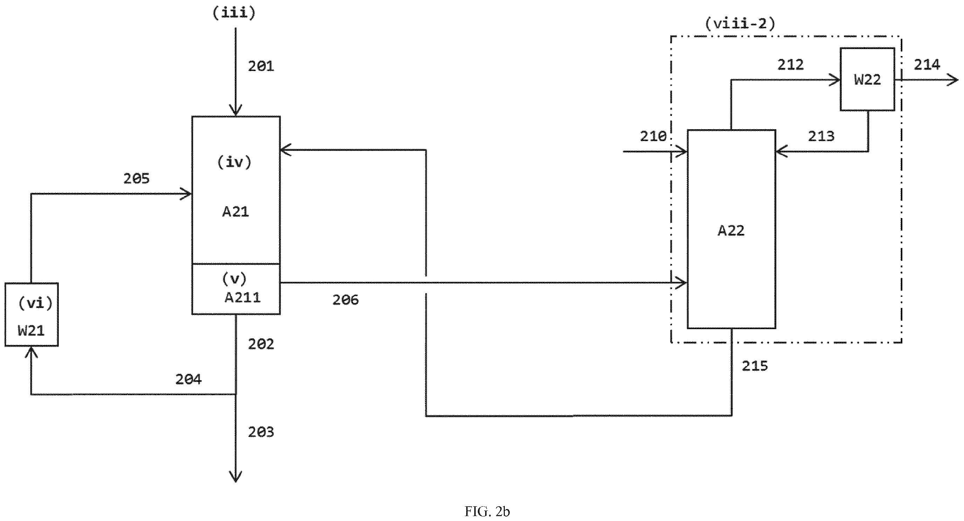

Geeignete Auswaschkolonnen sind dem Fachmann bekannt. Beispielhaft seinen Packungskolonnen und Bodenkolonnen genannt. Im Folgenden wird der Betrieb einer solchen Auswaschkolonne in Schritt (viii-2) anhand von **FIG. 2a** allgemein erläutert (FIG. 2a ist eine Ausschnittvergrößerung der Auswaschkolonne aus FIG. 2b; *die folgende Beschreibung ist jedoch nicht auf die* Ausführungsform *gemäß* *FIG. 2b* *beschränkt, sondern gilt für alle Ausführungsformen der Erfindung*):
Der von Isocyanat durch Auswaschung zu befreiende gasförmige Stoffstrom (206 in FIG. 2a) wird der Auswaschkolonne (A22) zugeleitet. Die dazu erforderliche Auswaschflüssigkeit wird am Kopf der Auswaschkolonne (A22) aufgegeben. Die Brüden (212) der Auswaschkolonne durchlaufen einen Kondensator *(Brüdenkondensator* W22). Selbstverständlich können auch mehrere solcher Kondensatoren, insbesondere zwei, in Reihe geschaltet, eingesetzt werden; allgemein gilt, dass wenn bei der Beschreibung der Erfindung von "einem" Apparat gesprochen wird und/oder in den Zeichnungen nur ein Apparat gezeigt ist, dies, sofern nicht anders vermerkt, Ausführungsformen, in denen zwei oder mehr Apparate für den jeweiligen Zweck eingesetzt werden, nicht ausschließt. Im Kondensator (W22) verflüssigte Anteile (213) werden auf den Kopf der Auswaschkolonne zurückgegeben und so in dieselbe rezykliert. Die nicht kondensierten Anteile (214), die überwiegend Chlorwasserstoffgas und Phosgen enthalten, werden, wie weiter unten beschrieben, bevorzugt zur Rückgewinnung von Chlorwaserstoff und Phosgen aufgearbeitet. Je größer der Gehalt an Isocyanat im zugeführten Stoffstrom (206) ist, desto größer muss der am Kopf der Auswaschkolonne zugeführte Strom an Auswaschflüssigkeit gewählt werden, und desto mehr Trennstufen werden für eine zuverlässige Rückhaltung benötigt. Die insgesamt in Schritt (viii-2) eingesetzte Auswaschflüssigkeit setzt sich aus dem Kondensat (213) des Kondensators (W22) und optional zusätzlich zugeführter Waschflüssigkeit (210) zusammen. Diese zusätzlich zugeführte Waschflüssigkeit (210) ist bevorzugt ein Lösungsmittelstrom, der ein organisches Lösungsmittel ausgewählt aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel enthält. Wie weiter unten noch näher erläutert wird, kann auf die zusätzlich zugeführte Waschflüssigkeit (210) in bestimmten Ausführungsformen der Erfindung verzichtet werden.

Es ist möglich, die in Schritt (v) erhaltene gasförmige, Chlorwasserstoff, nicht umgesetztes Phosgen, nicht verflüssigtes Isocyanat und - sofern vorhanden - verdampftes Lösungsmittel umfassende Phase unmittelbar, also ohne Durchlaufen der Kondensation in Schritt (viii-1), in die Auswaschkolonne aus Schritt (viii-2) zu leiten und dort mit Auswaschflüssigkeit in Kontakt zu bringen, wobei ein flüssiger, (mindestens) Auswaschflüssigkeit und Isocyanat umfassender Strom und ein gasförmiger, Chlorwasserstoff und Phosgen umfassender Strom erhalten werden. Dies ist in **FIG. 2b** gezeigt. Im Unterschied zu FIG. 1 beginnen alle Bezugszeichen mit der Ziffer 2 und haben ansonsten die gleiche Bedeutung wie dort. Der *apparative* Aufbau entspricht dem in FIG. 1 Gezeigten. Der im Sumpf der Auswaschkolonne A22 erhaltene Strom (215) - der flüssige, (mindestens) Auswaschflüssigkeit und Isocyanat umfassende Strom - enthält jedoch organische Lösungsmittel *nur in solchen Anteilen, dass die erfindungsgemäßen Anforderungen an den maximalen Lösungsmittelgehalt in der in* Schritt *(iv) insgesamt eingesetzten Quenchflüssigkeit erfüllt werden,* im Gegensatz zu dem in FIG. 1 gezeigten Sumpfstrom (115) aus der Auswaschkolonne A12, welcher, gemäß der Offenbarung der Anmeldung EP 1 935 875 A1, Absatz [0042], Lösungsmittel in Anteilen *von 80 bis 99,99 Massen-%* enthält. Strom 215 dient in dieser Ausfuhrungsform als zusätzliche Quenchflüssigkeit. Der Lösungsmittelgehalt des Stroms 215 wird eingestellt durch die Wahl der Zusammensetzung und des Mengenstroms der zugeführten Auswaschflüssigkeit (210) und durch die Wahl der Kondensationsleistung des Kondensators W22, welche für die Zusammensetzung und den Mengenstrom des Rückführstromes an rezykliertem Brüdenkondensat (213) wesentlich ist. Als Auswaschflüssigkeit (210) für die Auswaschkolonne A22 aus Schritt (viii-2) eignet sich ein Lösungsmittelstrom wie zuvor bei der allgemeinen Beschreibung der Auswaschkolonne definiert. Dabei ist selbstverständlich zu beachten, dass die Bedingungen - also insbesondere die Mengenströme an zu waschender Gasphase aus der Sammelzone (Schritt (v); 206), an zusätzlich zugeführter Auswaschflüssigkeit (210) und an zurückgeführtem Brüdenkondensat (213) -, so gewählt werden müssen, dass die erfindungsgemäßen Anforderungen im Hinblick auf den maximalen Lösungsmittelgehalt der Quenchflüssigkeit in Schritt (iv) (sowie des Sumpfstroms der Auswaschkolonne) eingehalten werden. Es ist auch möglich, dass die zusätzlich zugeführte Auswaschflüssigkeit (210) das herzustellende Isocyanat enthält, wobei das an dieser Stelle eingesetzte Isocyanat bevorzugt ein Teil des in Schritt (vii) gewonnenen Rein-Isocyanats ist. Es ist also grundsätzlich möglich, auf den Einsatz von Lösungsmittel als der Auswaschkolonne zusätzlich zugeführten Auswaschflüssigkeit (210) weitgehend, insbesondere vollständig zu verzichten. In jedem Fall wird der Gehalt an organischen Lösungsmitteln in diesem Strom (210) und/oder die absolute Menge des zugeführten Stroms (210) so gewählt, dass sich im Sumpfstrom (215) der Auswaschkolonne (A22) ein auf dessen Gesamtmasse bezogener Anteil an organischen Lösungsmitteln von 0,0 Massen-% bis 10 Massen-%, bevorzugt von 0,0 Massen-% bis 5,0 Massen-%, einstellt. Daneben enthält der Sumpfstrom das herzustellende Isocyanat.

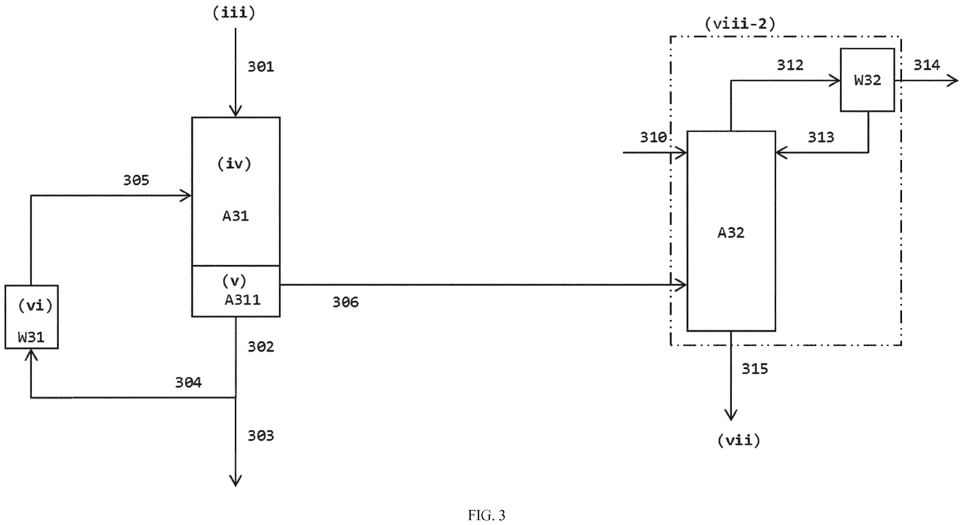

Das erfindungsgemäße Verfahren ermöglicht es in vorteilhafter Weise, den Sumpfstrom der Auswaschkolonne, anstatt ihn als Quenchflüssigkeit einzusetzen, in die Aufarbeitung gemäß Schritt (vii) zu führen, d. h. insbesondere in die Destillation zur Feinreinigung des Isocyanats in Schritt (vii-2), welche bevorzugt in einer einzigen Destillationskolonne, die insbesondere als Trennwandkolonne ausgestaltet sein kann, durchgeführt wird. Dies ist - in allgemeiner Weise - in **FIG. 3** gezeigt, wo im Unterschied zu FIG. 1 alle Bezugszeichen mit der Ziffer 3 beginnen und ansonsten die gleiche Bedeutung wie dort haben. Dann wird als in Schritt (iv) eingesetzter Quenchflüssigkeit nur die in Schritt (vi) erhaltene abgekühlte, ansonsten jedoch nicht in ihrer Zusammensetzung veränderte, flüssige Phase eingesetzt. Sofern der Feinreinigung des Isocyanats ein Destillationsschritt (vii-1) zur Lösungsmittelabtrennung vorgeschaltet ist - was bei den erfindungsgemäß geringen Lösungsmittelgehalten nicht zwingend erforderlich ist und daher bevorzugt unterbleibt - kann der Sumpfstrom 315 auch in diesen Schritt (vii-1) geführt werden. Das organische Lösungsmittel wird, sofern eingesetzt, bevorzugt aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel ausgewählt. Die genannten geringen Lösungsmittelgehalte von maximal 10 Massen-, bevorzugt maximal 5,0 Massen-% im Sumpfstrom der Auswaschkolonne lassen sich realisieren, indem als der Auswaschkolonne zusätzlich zugeführte Auswaschflüssigkeit (Strom 310) ein organisches Lösungsmittel (bevorzugt ohne Zugabe von Isocyanat) eingesetzt wird, dessen Mengenstrom entsprechend gering genug gewählt wird. Hierzu ist ein entsprechend großer Strom an rezykliertem Brüdenkondensat (313) erforderlich. Zweckmäßigerweise wird in dieser Ausführungsform bei der Inbetriebnahme der Auswaschkolonne (A32) in derselben Isocyanat vorgelegt, das dann über Strom 313 im Kreis geführt wird, sodass nur eine Zugabe geringer Mengen frischen Lösungsmittels über Strom (310) erforderlich ist. In dieser Ausführungsform ist es auch vorstellbar, auf den Strom (310) ganz zu verzichten und als Auswaschflüssigkeit ausschließlich rezykliertes Brüdenkondensat (313) einzusetzen. Auch in der Ausführungsform gemäß FIG. 2 kann auf die zusätzlich zugeführte Auswaschflüssigkeit (dort Strom 210) verzichtet werden, wenn der Strom an rezykliertem Brüdenkondensat (dort Strom 213) hinreichend groß ist.

Unabhängig davon, ob zusätzliche Auswaschflüssigkeit (210, 310) eingesetzt wird oder nicht, und unabhängig davon, ob das reyzklierte Brüdenkondensat (213, 313) Lösungsmittel enthält oder nicht, und unabhängig davon, ob der Sumpfstrom (215, 315) der Auswaschkolonne (A22, A32) in Schritt (vii) geführt oder als Quenchflüssigkeit eingesetzt wird, wird die Temperatur des als Auswaschflüssigkeit eingesetzten reyzklierten Brüdenkondensats (213, 313) bevorzugt auf eine Temperatur von maximal 100 °C, besonders bevorzugt von maximal 80 °C, ganz besonders bevorzugt von maximal 60 °C, eingestellt.

Die Kühlung des gasförmigen Reaktionsproduktgemisches in Schritt (iv) erfolgt in der Ausführungsform gemäß FIG. 3 nur durch Rezyklierung eines Teil der in Schritt (v) erhaltenen Flüssigphase aus dem Sammelbehälter. Der Mengenstrom des Stroms 304 und die Temperatur, mit welcher Strom 305 in die Quenche geführt wird, sind daher so zu wählen, dass eine ausreichende Kondensation des Reaktionsproduktgasgemisches in Schritt (iv) gewährleistet ist. Eine entsprechende Auslegung gehört zu den Routinetätigkeiten des Fachmanns.

Das erfindungsgemäße Verfahren ermöglicht infolge des geringen Lösungsmittelanteils der in Schritt (v) der Sammelzone entnommenen Gasphase apparative Vereinfachungen der Auswaschkolonne aus Schritt (viii-2). Insbesondere kann eine kleinere Ausführung der Auswaschkolonne (vorzugsweise mit verringertem Kolonnenquerschnitt) eingesetzt werden. Denkbar ist ebenfalls eine Verkleinerung von Peripheriegeräten wie Kondensatoren.

Unabhängig von der exakten Ausgestaltung des Schritts (viii-2) ist es bevorzugt, der Auswaschkolonne möglichst wenig frisches organisches Lösungsmittel (im Unterschied zu über eine Rezyklierung des Brüdenkondensats zurückgeführtes organisches Lösungsmittel) als Auswaschflüssigkeit zuzuführen. Insbesondere ist es bevorzugt, den Massenstrom an der Auswaschkolonne zugeführtem frischem organischem Lösungsmittel so einzustellen, dass dieser im Bereich von 20 % bis 100 %, besonders bevorzugt im Bereich von 50 % bis 100 %, des Massenstroms des gasförmigen Stroms des primären Amins aus Schritt (i) liegt.

Der in Schritt (viii-1) oder Schritt (viii-2) erhaltene gasförmige Strom wird bevorzugt in einem **Schritt (viii-3)** in an sich bekannter Weise von überschüssigem Phosgen befreit. Dies kann mittels einer Kühlfalle, Absorption in einem inerten Lösungsmittel (bevorzugt ausgewählt aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel) oder durch Adsorption und Hydrolyse an Aktivkohle erfolgen. Bevorzugt erfolgt dieser Schritt wie in WO2011/003532 A1 beschrieben; siehe insbesondere S. 11, Z. 31 bis S. 25, Z. 15. Der in der Phosgenabtrennung erhaltene, im Wesentlichen aus Chlorwasserstoff bestehende Gasstrom kann in der in der zuvor geschilderten Weise weiter verarbeitet werden.

Sofern im erfindungsgemäßen Verfahren überhaupt organische Lösungsmittel zum Einsatz kommen, wird in allen Schritten, in denen ein organisches Lösungsmittel eingesetzt wird, bevorzugt das gleiche organische Lösungsmittel eingesetzt, das insbesondere ausgewählt wird aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel. Besonders bevorzugt ist der Einsatz von ortho-Dichlorbenzol, und zwar insbesondere in Verbindung mit TDA als dem zu phosgenierenden primären Amin.

Das erfindungsgemäße Verfahren weist eine Reihe von Vorteilen auf, insbesondere:
a) Verringerung der internen Lösungsmittelkreisläufe;
b) Verkleinerung oder gar Eliminierung der Lösungsmitteldestillation gemäß Schritt (vii-1)
c) entsprechende Einsparung von einzubringender Verdampfungs-Energie;
d) Reduktion der die Sammelzone verlassenden Brüdenvolumina und dadurch Verkleinerung nachgeschalteter Apparate (z. B. der Isocyanat-Auswaschkolonne);
e) höhere Effizienz des Reaktorsumpfkühlers durch maximierten Wärmeübergang.

### Beispiele:

### Beispiel 1:

TDA wurde verdampft und erhitzt, und so ein Strom von 8,8 kg/h gasförmigen TDA's (380 °C, 1600 mbar) bereitgestellt *(Schritt (i)).* Parallel wurde ein Strom von 42,8 kg/h gasförmigen Phosgens (330 °C, 1600 mbar) bereitgestellt *(Schritt (ii)).* Vermischung, Reaktion und Quenche wurden in einem aufrecht angeordneten konisch-zylindrischen Reaktor, welcher Mischzone, Reaktionszone, Quenchzone und Sammelzone umfasste, durchgeführt. Zu diesem Zweck wurde der TDA-Strom mittels eines zentralen, vom Phosgen-Strom umgebenen Düsenrohrs von oben in den Reaktor eingeführt. Am Austritt des zentralen Düsenrohrs vermischte sich der Amin- mit dem einhüllenden Phosgenstrom *(Schritt (iii))* unter adiabatischen Bedingungen. Das so erhaltene Reaktionsgemisch strömte (weiterhin unter adiabatischen Bedingungen) durch die Reaktionszone *(Schritt (iii))* nach unten und wurde in einer einstufig ausgestalteten Quenche durch Eindüsen von Quenchflüssigkeit bestehend aus zurückgeführtem Reaktorsumpf abgekühlt *(Schritt iv)).* Das in der Quenche gebildete Gemisch aus Reaktionsproduktgemisch und Quenchflüssigkeit strömte in den Reaktorsumpfbehälter (die *Sammelzone; Schritt (v)).* Erfindungsgemäß wurden der Sammelzone 12,1 kg/h des flüssigen Reaktorsumpfes, der 98 Massen-% TDI enthielt, entnommen. 97 % dieses entnommenen Reaktorsumpfes wurden über einen Kühler auf 150 °C abgekühlt und in die einstufig ausgestaltete Quenchezone des Reaktors als (einzige) Quenchflüssigkeit eingedüst (*Schritt (vi)*). Der restliche Teil des der Sammelzone entnommenen flüssigen Reaktorsumpfes wurde zu TDI aufgearbeitet (*Schritt vii)*). Nach 92 Stunden Laufzeit wurden die Eduktdosierung gestoppt und der Reaktor abgekühlt. Die Begutachtung des Reaktors und der Kühlzone ergab, dass diese trotz des weitgehend lösungsmittelfreien Betriebes praktisch frei von festen Rückständen waren.

Das den Reaktor verlassende Prozessgas enthaltend Chlorwasserstoff, nicht umgesetztes Phosgen und Anteile nicht verflüssigten Isocyanats eignet sich zur Rückgewinnung der Isocyanat-Anteile in einer lösungsmittelarm betriebenen Auswaschkolonne im Sinne des Schritts (viii-2). Aufgrund des geringen Lösungsmittelanteils ist eine Rückführung des ausgewaschenen lösungsmittelhaltigen TDI in den Reaktor als Quenchflüssigkeit nicht erforderlich.

Die Ausbeute des Prozesses lag bei 98,2 %.

### Beispiel 2:

Es wurde mit Ausnahme der folgenden Unterschiede verfahren wie in Beispiel 1 beschrieben:

| | |
|---|---|
| Mengenstrom gasförmiges TDA (380 °C, 1600 mbar) : | 12000 kg/h |
| Mengenstrom gasförmiges Phosgen (320 °C, 1700 mbar): | 6000 kg/h |
| Abkühlung des in die Quenche rezyklierten Reaktorsumpfes: | 160 °C |

Auch in diesem Beispiel betrug die TDI-Konzentration in der TDI-Rohware 98 Massen-%.

Die Ausbeute des Prozesses lag bei 97,8 %.

### Beispiel 3 (Vergleichsbeispiel):

Es wurde mit Ausnahme der folgenden Unterschiede verfahren wie in Beispiel 2 beschrieben:
Die Quenchezone war zweistufig ausgestaltet, wobei der oberen Quenchestufe eine 10%ige Lösung von TDI in ortho-Dichlorbenzol zugeführt wurde und der unteren Quenchestufe der auf 160 °C gekühlte umgepumpte Reaktorsumpf mit einer TDI-Konzentration von 35 Massen-% in ortho-Dichlorbenzol zugeführt wurde.

Die Ausbeute des Prozesses lag bei 97,6 %.

In den erfindungsgemäßen Beispielen konnte die TDI-Konzentration in der Rohware erfolgreich wesentlich erhöht werden, ohne dass damit Nachteile verbunden waren.

## Patentansprüche

1. Verfahren zur Herstellung eines Isocyanats durch Phosgenierung des korrespondierenden primären Amins in der Gasphase umfassend die Schritte:
(i) Bereitstellung eines gasförmigen Stroms eines primären Amins;
(ii) Bereitstellung eines gasförmigen Phosgenstroms;
(iii) Vermischen des gasförmigen Stroms des primären Amins aus Schritt (i) und des gasförmigen Phosgenstroms aus Schritt (ii) zu einem gasförmigen Reaktionsgemisch unter Einhaltung eines auf primäre Aminogruppen bezogenen stöchiometrischen Überschusses an Phosgen in einer Mischzone und Führen des so erhaltenen gasförmigen Reaktionsgemisches durch eine Reaktionszone zur Umsetzung des primären Amins mit Phosgen unter Erhalt eines gasförmigen Reaktionsproduktgemisches;
(iv) Abkühlen des nach Durchlaufen der Reaktionszone aus Schritt (iii) erhaltenen gasförmigen Reaktionsproduktgemisches durch Inkontaktbringen mit mindestens einem Strom einer Quenchflüssigkeit in einer Quenchzone, wobei die insgesamt eingesetzte Quenchflüssigkeit, bezogen auf ihre Gesamtmasse, organische Lösungsmittel in einem Anteil von maximal 25 Massen-% umfasst, wobei der Rest zu 100 Massen-% aus mindestens dem herzustellenden Isocyanat besteht, unter Erhalt eines Gemisches aus Reaktionsproduktgemisch und Quenchflüssigkeit;
(v) Führen des in Schritt (iv) erhaltenen Gemisches aus Reaktionsproduktgemisch und Quenchflüssigkeit in eine Sammelzone zur Phasentrennung, wobei eine flüssige, Isocyanat umfassende Phase und eine gasförmige, Chlorwasserstoff, nicht umgesetztes Phosgen und nicht verflüssigtes Isocyanat umfassende Phase der Sammelzone entnommenen werden;
(vi) Abkühlen eines Teils der in Schritt (v) der Sammelzone entnommenen flüssigen, Isocyanat umfassenden Phase, und Einsatz der so erhaltenen abgekühlten, ansonsten jedoch nicht in ihrer Zusammensetzung veränderten, flüssigen Phase in Schritt (iv) als Bestandteil, gegebenenfalls als alleinigen Bestandteil, der Quenchflüssigkeit;
(vii) Aufarbeitung des nicht in Schritt (vi) abgekühlten und in Schritt (iv) eingesetzten Teils der in Schritt (v) der Sammelzone entnommenen flüssigen, Isocyanat umfassenden Phase zur Gewinnung des Isocyanats;
(viii-1) optional, teilweises Kondensieren der in Schritt (v) erhaltenen gasförmigen, Chlorwasserstoff, nicht umgesetztes Phosgen und nicht verflüssigtes Isocyanat umfassenden Phase durch Abkühlung unter Erhalt eines flüssigen, Isocyanat umfassenden Stroms und eines gasförmigen, Chlorwasserstoff, Phosgen und nicht verflüssigtes Isocyanat umfassenden Stroms;
(viii-2) Leiten
• entweder, bei Durchführung von Schritt (viii-1), des in Schritt (viii-1) erhaltenen gasförmigen, Chlorwasserstoff, Phosgen und nicht verflüssigtes Isocyanat umfassenden Stroms,
• oder, wenn Schritt (viii-1) nicht durchgeführt wird, der in Schritt (v) erhaltenen gasförmigen, Chlorwasserstoff, nicht umgesetztes Phosgen und nicht verflüssigtes Isocyanat umfassenden Phase,
in eine Auswaschkolonne, in welcher Isocyanat mit einer Auswaschflüssigkeit unter Erhalt eines flüssigen, Auswaschflüssigkeit und Isocyanat umfassenden Stroms und eines gasförmigen, Chlorwasserstoff und Phosgen umfassenden Stroms ausgewaschen wird und der dabei erhaltene gasförmige, Chlorwasserstoff und Phosgen umfassende Strom in einem der Auswaschkolonne nachgeschalteten Kondensator teilweise kondensiert wird, wobei sich die in Schritt (viii-2) insgesamt eingesetzte Auswaschflüssigkeit aus dem in dem der Auswaschkolonne nachgeschalteten Kondensator erhaltenen Kondensat und optional zusätzlich zugeführter Auswaschflüssigkeit zusammensetzt, wobei über die zusätzlich zugeführte Auswaschflüssigkeit organisches Lösungsmittel allenfalls in einer solchen Menge in die Auswaschkolonne eingetragen wird, dass sich in dem in der Auswaschkolonne erhaltenen, flüssigen, Auswaschflüssigkeit und Isocyanat umfassenden Strom ein auf dessen Gesamtmasse bezogener Anteil an organischen Lösungsmitteln von 0,0 Massen-% bis 10 Massen-% einstellt.

2. Verfahren gemäß Anspruch 1, bei welchem das Inkontaktbringen des gasförmigen Reaktionsproduktgemisches mit der Quenchflüssigkeit in Schritt (iv) durch Eindüsen der Quenchflüssigkeit in das gasförmige Reaktionsproduktgemisch erfolgt.

3. Verfahren gemäß Anspruch 2, bei welchem das Inkontaktbringen des gasförmigen Reaktionsproduktgemisches mit der Quenchflüssigkeit in Schritt (iv) einstufig erfolgt.

4. Verfahren gemäß einem der vorstehenden Ansprüche, bei welchem sich die in Schritt (viii-2) insgesamt eingesetzte Auswaschflüssigkeit aus dem in der Auswaschkolonne nachgeschalteten Kondensator erhaltenen Kondensat und zusätzlich zugeführter Auswaschflüssigkeit zusammensetzt, wobei diese zusätzlich zugeführte Auswaschflüssigkeit ein Lösungsmittelstrom ist, der ausschließlich ein organisches Lösungsmittel enthält, das ausgewählt ist aus der Gruppe bestehend aus aliphatischen Kohlenwasserstoffen, aromatischen Kohlenwasserstoffen ohne Halogensubstitution, aromatischen Kohlenwasserstoffen mit Halogensubstitution und Mischungen der vorgenannten Lösungsmittel.

5. Verfahren gemäß Anspruch 4, bei welchem der der Massenstrom der der Auswaschkolonne zusätzlich zugeführten Auswaschflüssigkeit so eingestellt wird, dass dieser im Bereich von 20 % bis 100 % des Massenstroms des gasförmigen Stroms des primären Amins aus Schritt (i) liegt.

6. Verfahren gemäß einem der vorstehenden Ansprüche, bei welchem der in der Auswaschkolonne erhaltene, flüssige, Auswaschflüssigkeit und Isocyanat umfassende Strom der Aufarbeitung in Schritt (vii) zugeführt wird.

7. Verfahren gemäß einem der vorstehenden Ansprüche, umfassend Schritt (viii-1), bei welchem die in Schritt (iv) eingesetzte Quenchflüssigkeit zusätzlich zu der in Schritt (vi) erhaltenen abgekühlten, ansonsten jedoch nicht in ihrer Zusammensetzung veränderten, flüssigen Phase, den in Schritt (viii-1) erhaltenen flüssigen, Isocyanat umfassenden Strom umfasst.

8. Verfahren gemäß einem der vorstehenden Ansprüche, bei welchem die in Schritt (iv) eingesetzte Quenchflüssigkeit zusätzlich zu der in Schritt (vi) erhaltenen abgekühlten, ansonsten jedoch nicht in ihrer Zusammensetzung veränderten, flüssigen Phase, den in Schritt (viii-2) erhaltenen flüssigen, Auswaschflüssigkeit und Isocyanat umfassenden Strom umfasst.

9. Verfahren gemäß einem der Ansprüche 1 bis 6, bei welchem die in Schritt (iv) eingesetzte Quenchflüssigkeit neben der in Schritt (vi) erhaltenen abgekühlten, ansonsten jedoch nicht in ihrer Zusammensetzung veränderten, flüssigen Phase keine weiteren Bestandteile enthält.

10. Verfahren gemäß einem der vorstehenden Ansprüche, bei welchem zur Gewinnung des Isocyanats in Schritt (vii) die flüssige, Isocyanat umfassenden Phase ohne zwischengeschaltete Lösungsmittelkolonne einer destillativen Reinigung zugeführt wird.

11. Verfahren gemäß Anspruch 10, bei welchem die destillative Reinigung in einer einzigen Destillationskolonne, die insbesondere als Trennwandkolonne ausgestaltet ist, durchgeführt wird.

12. Verfahren gemäß einem der vorstehenden Ansprüche, bei welchem jeder einzelne in Schritt (iv) eingesetzte Strom an Quenchflüssigkeit, bezogen auf seine Gesamtmasse, organische Lösungsmittel in einem Anteil von maximal 25 Massen-% umfasst.

13. Verfahren gemäß einem der vorstehenden Ansprüche, bei welchem das primäre Amin ausgewählt ist aus der Gruppe bestehend aus Toluylendiamin, Diphenylmethandiamin, Xylylendiamin, 1,5-Pentandiamin, 1,6-Hexamethylendiamin, Isophorondiamin, Diaminodicyclohexylmethan und Mischungen der vorgenannten Verbindungen.

14. Verfahren gemäß Anspruch 13, bei welchem das primäre Amin Toluylendiamin ist.

15. Verfahren gemäß einem der vorstehenden Ansprüche, bei welchem in Schritt (viii-2) über die zusätzlich zugeführte Auswaschflüssigkeit organisches Lösungsmittel allenfalls in einer solchen Menge in die Auswaschkolonne eingetragen wird, dass sich in dem in der Auswaschkolonne erhaltenen, flüssigen, Auswaschflüssigkeit und Isocyanat umfassenden Strom ein auf dessen Gesamtmasse bezogener Anteil an organischen Lösungsmitteln von 0,0 Massen-% bis 5,0 Massen-% einstellt

## Claims

1. Process for preparing an isocyanate by phosgenating the corresponding primary amine in the gas phase, comprising the steps of:
(i) providing a gaseous stream of a primary amine;
(ii) providing a gaseous phosgene stream;
(iii) mixing the gaseous stream of the primary amine from step (i) and the gaseous phosgene stream from step (ii) to give a gaseous reaction mixture while maintaining a stoichiometric excess of phosgene relative to primary amino groups in a mixing zone and conducting the gaseous reaction mixture thus obtained through a reaction zone for reaction of the primary amine with phosgene to obtain a gaseous reaction product mixture;
(iv) cooling the gaseous reaction product mixture obtained after passage through the reaction zone from step (iii) by contacting with at least one stream of a quench liquid in a quench zone, where the total quench liquid used, based on its total mass, comprises organic solvents in a proportion of at most 25% by mass, where the remainder to 100% by mass consists of at least the isocyanate to be prepared, to obtain a mixture of reaction product mixture and quench liquid;
(v) conducting the mixture of reaction product mixture and quench liquid obtained in step (iv) into a collecting zone for phase separation, where a liquid phase comprising isocyanate and a gaseous phase comprising hydrogen chloride, unconverted phosgene and unliquefied isocyanate are withdrawn from the collecting zone;
(vi) cooling a portion of the liquid phase comprising isocyanate withdrawn from the collecting zone in step (v), and using the cooled liquid phase of otherwise unchanged composition thus obtained in step (iv) as a constituent, optionally as the sole constituent, of the quench liquid;
(vii) working-up the portion of the liquid phase comprising isocyanate withdrawn from the collecting zone in step (v) that has not been cooled down in step (vi) and used in step (iv) to obtain the isocyanate;
(viii-1) optionally partly condensing the gaseous phase comprising hydrogen chloride, unconverted phosgene and unliquefied isocyanate which is obtained in step (v) by cooling to obtain a liquid stream comprising isocyanate and a gaseous stream comprising hydrogen chloride, phosgene and unliquefied isocyanate;
(viii-2) guiding either
• when step (viii-1) is conducted, the gaseous stream comprising hydrogen chloride, phosgene and unliquefied isocyanate which is obtained in step (viii-1) or
• when step (viii-1) is not conducted, the gaseous phase comprising hydrogen chloride, unconverted phosgene and unliquefied isocyanate which is obtained in step (v)
into a scrubbing column in which isocyanate is scrubbed out with a scrubbing liquid to obtain a liquid stream comprising scrubbing liquid and isocyanate and a gaseous stream comprising hydrogen chloride and phosgene, and the gaseous stream comprising hydrogen chloride and phosgene obtained here is partly condensed in a condenser downstream of the scrubbing column, where the scrubbing liquid used overall in step (viii-2) is composed of the condensate obtained in the condenser downstream of the scrubbing column and optionally additionally supplied scrubbing liquid, where organic solvent is introduced into the scrubbing column via the additionally supplied scrubbing liquid at most in such an amount that a proportion of organic solvents of 0.0% by mass to 10% by mass is established in the liquid stream comprising scrubbing liquid and isocyanate which is obtained in the scrubbing column, based on the total mass thereof.

2. Process according to Claim 1, in which the gaseous reaction product mixture is contacted with the quench liquid in step (iv) by injecting the quench liquid into the gaseous reaction product mixture.

3. Process according to Claim 2, in which the contacting of the gaseous reaction product mixture with the quench liquid in step (iv) is effected in one stage.

4. Process according to any of the preceding claims, in which the scrubbing liquid used overall in step (viii-2) is composed of the condensate obtained in the condenser downstream of the scrubbing column and additionally supplied scrubbing liquid, where this additionally supplied scrubbing liquid is a solvent stream containing exclusively an organic solvent selected from the group consisting of aliphatic hydrocarbons, aromatic hydrocarbons without halogen substitution, aromatic hydrocarbons with halogen substitution and mixtures of the aforementioned solvents.

5. Process according to Claim 4, in which the mass flow rate of the scrubbing liquid additionally supplied to the scrubbing column is adjusted such that it is within the range from 20% to 100% of the mass flow rate of the gaseous stream of the primary amine from step (i).

6. Process according to any of the preceding claims, in which the liquid stream comprising scrubbing liquid and isocyanate which is obtained in the scrubbing column is sent to the workup in step (vii) .

7. Process according to any of the preceding claims, comprising step (viii-1), in which the quench liquid used in step (iv), in addition to the cooled liquid phase of otherwise unchanged composition that has been obtained in step (vi), comprises the liquid stream comprising isocyanate that has been obtained in step (viii-1).

8. Process according to any of the preceding claims, in which the quench liquid used in step (iv), in addition to the cooled liquid phase of otherwise unchanged composition that has been obtained in step (vi), comprises the liquid stream comprising scrubbing liquid and isocyanate that has been obtained in step (viii-2).

9. Process according to any of Claims 1 to 6, in which the quench liquid used in step (iv), aside from the cooled liquid phase of otherwise unchanged composition that has been obtained in step (vi), does not comprise any further constituents.

10. Process according to any of the preceding claims, in which the isocyanate is obtained in step (vii) by supplying the liquid phase comprising isocyanate to a distillative purification without an intermediate solvent column.

11. Process according to Claim 10, in which the distillative purification is performed in a single distillation column which is especially configured as a dividing wall column.

12. Process according to any of the preceding claims, in which every individual stream of quench liquid used in step (iv), based on its total mass, comprises organic solvents in a proportion of at most 25% by mass.

13. Process according to any of the preceding claims, in which the primary amine is selected from the group consisting of tolylenediamine, diphenylmethanediamine, xylylenediamine, pentane-1,5-diamine, hexamethylene-1,6-diamine, isophoronediamine, diaminodicyclohexylmethane and mixtures of the aforementioned compounds.

14. Process according to Claim 13, in which the primary amine is tolylenediamine.

15. Process according to any of the preceding claims, in which, in step (viii-2), organic solvent is introduced into the scrubbing column via the additionally supplied scrubbing liquid at most in such an amount that a proportion of organic solvents of 0.0% by mass to 5.0% by mass is established in the liquid stream comprising scrubbing liquid and isocyanate which is obtained in the scrubbing column, based on the total mass thereof.

## Revendications

1. Procédé de préparation d'un isocyanate par phosgénation de l'amine primaire correspondante en phase gazeuse comprenant les étapes :
(i) mise à disposition d'un flux gazeux d'une amine primaire ;
(ii) mise à disposition d'un flux gazeux de phosgène ;
(iii) mélange du flux gazeux de l'amine primaire de l'étape (i) et du flux gazeux de phosgène de l'étape (ii) pour donner un mélange de réaction gazeux en respectant un excès stoechiométrique de phosgène par rapport aux groupes amino primaire dans une zone de mélange et conduite du mélange de réaction gazeux ainsi obtenu à travers une zone de réaction pour la transformation de l'amine primaire avec du phosgène avec obtention d'un mélange gazeux de produits de réaction ;
(iv) refroidissement du mélange gazeux de produits de réaction obtenu après passage de la zone de réaction de l'étape (iii) par mise en contact avec au moins un flux d'un liquide de trempe dans une zone de trempe, dans lequel le liquide de trempe utilisé au total comprend, par rapport à sa masse totale, des solvants organiques en une proportion maximale de 25 % en masse, dans lequel le reste jusqu'à 100 % en masse est constitué au moins de l'isocyanate à préparer, avec obtention d'un mélange de mélange de produits de réaction et de liquide de trempe ,
(v) conduite du mélange obtenu dans l'étape (iv) de mélange de produits de réaction et de liquide de trempe dans une zone de collecte pour une séparation de phases, une phase liquide comprenant de l'isocyanate et une phase gazeuse comprenant du chlorure d'hydrogène, du phosgène n'ayant pas réagi et de l'isocyanate non liquéfié étant prélevées de la zone de collecte ;
(vi) refroidissement d'une partie de la phase liquide comprenant de l'isocyanate prélevée de la zone de collecte dans l'étape (v), et utilisation de la phase liquide refroidie ainsi obtenue, mais cependant non modifiée dans sa composition, dans l'étape (iv) en tant qu'ingrédient, éventuellement en tant que seul ingrédient, du liquide de trempe ;
(vii) traitement de la partie de la phase liquide comprenant de l'isocyanate, prélevée de la zone de collecte dans l'étape (v), non refroidie dans l'étape (vi) et utilisée dans l'étape (iv) pour l'obtention de l'isocyanate ;
(viii-1) éventuellement, condensation partielle de la phase gazeuse comprenant du chlorure d'hydrogène, du phosgène n'ayant pas réagi et de l'isocyanate non liquéfié, obtenue dans l'étape (v), par refroidissement avec obtention d'un flux liquide comprenant de l'isocyanate et d'un flux gazeux contenant du chlorure d'hydrogène, du phosgène et de l'isocyanate non liquéfié ;
(viii-2) conduite
• soit, lors de la réalisation de l'étape (viii-1), du flux gazeux contenant du chlorure d'hydrogène, du phosgène et de l'isocyanate non liquéfié obtenu dans l'étape (viii-1),
• soit, lorsque l'étape (viii-1) n'est pas réalisée, de la phase gazeuse contenant du chlorure d'hydrogène, du phosgène n'ayant pas réagi et de l'isocyanate non liquéfié, obtenue dans l'étape (v),
dans une colonne de lavage, dans laquelle de l'isocyanate est lavé avec un liquide de lavage avec obtention d'un flux liquide comprenant du liquide de lavage et de l'isocyanate et d'un flux gazeux comprenant du chlorure d'hydrogène et du phosgène et le flux gazeux ainsi obtenu comprenant du chlorure d'hydrogène et du phosgène est partiellement condensé dans un condenseur monté en aval de la colonne de lavage, le liquide de lavage utilisé au total dans l'étape (viii-2) étant composé du condensat obtenu dans le condenseur monté en aval de la colonne de lavage et de liquide de lavage éventuellement alimenté en plus, du solvant organique étant éventuellement introduit, sur le liquide de lavage alimenté en plus, en une telle quantité dans la colonne de lavage, que dans le flux liquide, comprenant du liquide de lavage et de l'isocyanate, obtenu dans la colonne de lavage, une proportion en solvant organique, par rapport à sa masse totale, s'ajuste de 0,0 % en masse à 10 % en masse.

2. Procédé selon la revendication 1, dans lequel la mise en contact du mélange gazeux de produits de réaction avec le liquide de trempe dans l'étape (iv) est réalisée par injection du liquide de trempe dans le mélange gazeux de produits de réaction.

3. Procédé selon la revendication 2, dans lequel la mise en contact du mélange gazeux de produits de réaction avec le liquide de trempe dans l'étape (iv) est réalisée en une étape.

4. Procédé selon l'une quelconque des revendications précédentes, dans lequel le liquide de lavage utilisé au total dans l'étape (viii-2) est composé du condensat obtenu dans le condenseur monté en aval de la colonne de lavage et de liquide de lavage alimenté en plus, ce liquide de lavage alimenté en plus étant un flux de solvant qui contient exclusivement un solvant organique qui est choisi dans le groupe constitué par des hydrocarbures aliphatiques, des hydrocarbures aromatiques sans substitution par halogène, des hydrocarbures aromatiques avec substitution par halogène et des mélanges des solvants mentionnés.

5. Procédé selon la revendication 4, dans lequel le flux de masse du liquide de lavage alimenté en plus à la colonne de lavage est ajusté de telle manière que celui-ci se situe dans la plage de 20 % à 100 % du flux de masse du flux gazeux de l'amine primaire de l'étape (i).

6. Procédé selon l'une quelconque des revendications précédentes, dans lequel le flux liquide comprenant du liquide de lavage et de l'isocyanate obtenu dans la colonne de lavage est alimenté au traitement dans l'étape (vii).

7. Procédé selon l'une quelconque des revendications précédentes, comprenant l'étape (viii-1), dans laquelle le liquide de trempe utilisé dans l'étape (iv) comprend en plus de la phase liquide refroidie obtenue dans l'étape (vi), mais cependant non modifiée dans sa composition, le flux liquide comprenant de l'isocyanate obtenu dans l'étape (viii-1).

8. Procédé selon l'une quelconque des revendications précédentes, dans lequel le liquide de trempe utilisé dans l'étape (iv) comprend en plus de la phase liquide refroidie obtenue dans l'étape (vi), mais cependant non modifiée dans sa composition, le flux liquide comprenant du liquide de lavage et de l'isocyanate, obtenu dans l'étape (viii-2).

9. Procédé selon l'une quelconque des revendications 1 à 6, dans lequel le liquide de trempe utilisé dans l'étape (iv), outre la phase liquide refroidie obtenue dans l'étape (vi), mais cependant non modifiée dans sa composition, ne contient aucun autre ingrédient.

10. Procédé selon l'une quelconque des revendications précédentes, dans lequel pour l'obtention de l'isocyanate dans l'étape (vii), la phase liquide comprenant de l'isocyanate est alimentée à une purification par distillation, sans colonne de solvant intermédiaire.

11. Procédé selon la revendication 10, dans lequel la purification par distillation est mise en œuvre dans une seule colonne de distillation qui est conçue en particulier comme colonne à parois de séparation.

12. Procédé selon l'une quelconque des revendications précédentes, dans lequel chaque flux de liquide de trempe respectif utilisé dans l'étape (iv) comprend, par rapport à sa masse totale, des solvants organiques en une proportion maximale de 25 % en masse.

13. Procédé selon l'une quelconque des revendications précédentes, dans lequel l'amine primaire est choisie dans le groupe constitué par une toluylènediamine, une diphénylméthanediamine, une xylylènediamine, la 1,5-pentanediamine, la 1,6-hexaméthylènediamine, l'isophoronediamine, un diaminodicyclohexylméthane et des mélanges des composés mentionnés précédemment.

14. Procédé selon la revendication 13, dans lequel l'amine primaire est une toluylènediamine.

15. Procédé selon l'une quelconque des revendications précédentes, dans lequel dans l'étape (viii-2) du solvant organique est éventuellement introduit, sur le liquide de lavage alimenté en plus, en une telle quantité dans la colonne de lavage, que dans le flux liquide, comprenant du liquide de lavage et de l'isocyanate, obtenu dans la colonne de lavage, une proportion en solvant organique, par rapport à sa masse totale, s'ajuste de 0,0 % en masse à 5,0 % en masse.
